# EUROPÄISCHE PATENTSCHRIFT

(11) **EP 1 957 603 B1**
(45) Veröffentlichungstag und Bekanntmachung des Hinweises auf die Patenterteilung: **14.10.2015**
(21) Anmeldenummer: 06818609.7
(22) Anmeldetag: 17.11.2006
(51) Int. Cl.: C09K 11/06, H05B 33/14, C07C 211/54

(54) **ORGANISCHE ELEKTROLUMINESZENZVORRICHTUNGEN**
ORGANIC ELECTROLUMINESCENT DEVICES
DISPOSITIFS ORGANIQUES ÉLECTROLUMINESCENTS

(30) Priorität: 08.12.2005 DE 102005058557
(43) Veröffentlichungstag der Anmeldung: 20.08.2008
(73) Patentinhaber: Merck Patent GmbH, 64293 Darmstadt (DE)
(72) Erfinder: VESTWEBER, Horst, 34630 Gilersberg-Winterscheid (DE); STOESSEL, Philipp, 60487 Frankfurt am Main (DE); HEIL, Holger, 64295 Darmstadt (DE)
(86) Internationale Anmeldenummer: PCT/EP2006/011026
(87) Internationale Veröffentlichungsnummer: WO 2007/065547

(56) Entgegenhaltungen:
- EP-A- 1 533 289
- EP-A- 1 533 290
- EP-A- 1 553 154
- EP-A1- 0 866 110
- EP-A1- 1 167 488
- WO-A-2006/000389
- US-A- 5 998 046
- WANG X M ET AL: "SYNTHESIS OF NEW MULTIBRANCH CHROMOPHORES WITH STRON LIGHT-EMITTING IN SOLUTION AND IN PMMA FILM" CHINESE CHEMICAL LETTERS, XX, XX, Bd. 14, 2003, Seiten 1135-1138, XP008073607 ISSN: 1001-8417

## Beschreibung

Die vorliegende Erfindung betrifft Mischungen organischer Materialien, die Verwendung dieser Mischungen in organischen Elektrolumineszenzvorrichtungen und organische Elektrolumineszenzvorrichtungen enthaltend diese Mischungen.

Der allgemeine Aufbau organischer Elektrolumineszenzvorrichtungen, die zur Emission von Licht im sichtbaren Spektralbereich befähigt sind und die halbleitende organische Verbindungen enthalten, ist beispielsweise in US 4539507, US 5151629, EP 0676461 und WO 98/27136 beschrieben.

Allerdings zeigen diese Vorrichtungen immer noch erhebliche Probleme, die für die Verwendung in hochwertigen Vollfarbdisplays einer dringenden Verbesserung bedürfen:
1. Die operative Lebensdauer ist insbesondere bei blauer Emission immer noch gering, so dass bis dato nur einfache Anwendungen kommerziell realisiert werden konnten.
2. Die Systeme mit den bislang besten Lebensdauern zeigen hellblaue oder blaugrüne, aber keine tiefblaue Emission und sind daher für hochwertige Anwendungen ungeeignet.
3. Viele blau emittierende Emitter, die sowohl aromatische Amine, wie auch Doppelbindungssysteme enthalten, sind thermisch nicht stabil und zersetzen sich beim Sublimieren oder beim Aufdampfen. Dadurch ist die Verwendung dieser Systeme nicht bzw. nur unter großen Verlusten und mit hohem technischen Aufwand möglich.
4. Insbesondere bei der Kombination von guter Lebensdauer und tiefblauer Emission besteht noch deutlicher Verbesserungsbedarf.

In JP 04-184892 werden Distilbenamine, Tristilbenamine und weitere Stilbenderivate als emittierende Verbindungen für OLEDs beschrieben. Diese Verbindungen werden in der emittierenden Schicht als Reinsubstanz eingesetzt. Die Emissionsfarbe ist nicht beschrieben. Ebenso sind weder die Effizienz noch die Lebensdauer der OLED aufgeführt. Diese Stilbenamine zeigen im vorgeschlagenen Deviceaufbau blaugrüne bzw. grüne Emission und sind nicht zur Erzeugung tiefblauer Emission geeignet.

In EP 1167488 wird die Kombination von Monostyrylaminen, Distyrylaminen, Tristyrylaminen bzw. Tetrastyrylaminen zusammen mit bestimmten Dianthracenderivaten bzw. mit Anthracenderivaten, welche mit kondensierten Aromaten oder mit Arylgruppen mit 12 oder mehr Kohlenstoffatomen substituiert sind, vorgeschlagen. Dies wird nur an linearen Styrylaminen ausgeführt. Ebenso wurden die weiteren Entwicklungen des Hostmaterials nur für lineare Styrylamine optimiert (z. B. WO 04/013073, WO 04/016575, WO 04/018587). Es werden gute Lebensdauern bei blauer Emission genannt; Farbkoordinaten sind keine angegeben. Hier erhält man statt tiefblauer nur grünblaue Emission. Es ist nicht offensichtlich, wie mit diesen Materialkombinationen blaue, insbesondere tiefblaue Emission erzeugt werden kann. Weiterhin sind die im Stand der Technik verwendeten emittierenden Verbindungen thermisch instabil und lassen sich nicht unzersetzt verdampfen, was einen hohen technischen Aufwand für die Aufdampfung erfordert und somit einen deutlichen technischen Nachteil darstellt.

Es war daher die Aufgabe der vorliegenden Erfindung, hierfür Verbesserungen anzubieten, insbesondere Systeme mit verbesserter thermischer Stabilität und tiefblauer Emissionsfarbe bei vergleichbar guter oder verbesserter Lebensdauer.

Überraschend wurde gefunden, dass organische Elektrolumineszenzvorrichtungen, die eine Kombination aus Tristilbenaminen und bestimmten, im Folgenden aufgeführten, Anthracenderivaten in der emittierenden Schicht enthalten, deutliche Verbesserungen gegenüber dem Stand der Technik aufweisen. Mit dieser Materialkombination können vergleichbare oder verbesserte Lebensdauern bei deutlich verbesserter, tiefblauer Emissionsfarbe erhalten werden. Außerdem lassen sich die Emissionsmaterialien im Gegensatz zu den Materialien, die gemäß dem Stand der Technik üblicherweise in Kombination mit Anthracenderivaten verwendet werden, ohne merkliche Zersetzung sublimieren und aufdampfen und sind daher deutlich leichter zu handhaben als Emitter, die gemäß dem Stand der Technik verwendet werden. Dieses Ergebnis ist insbesondere daher überraschend, da aus dem oben genannten Stand der Technik bekannt ist, dass die Emitter alleine grüne oder grünblaue Emission zeigen und dass lineare Styrylamine in Anthracen-Hostmaterialien ebenfalls nur grünblaue Emission zeigen. Dass Tristilbenamine in Anthracen-Hostmaterialien tiefblaue Emission zeigen, ist daher ein unerwartetes und nicht vorhersehbares Ergebnis. Diese Materialmischungen und deren Verwendung in OLEDs sind daher der Gegenstand der vorliegenden Erfindung.

Gegenstand der Erfindung sind organische Elektrolumineszenzvorrichtungen, enthaltend Anode, Kathode und mindestens eine organische Schicht, dadurch gekennzeichnet, dass die organische Schicht die folgenden Komponenten enthält:
a) mindestens eine Verbindung der Formel (1), 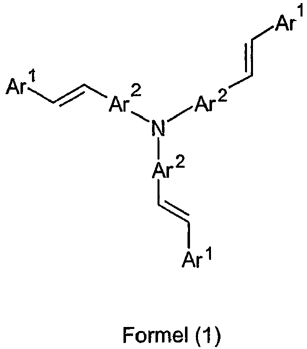 wobei für die verwendeten Symbole gilt:
   - Ar¹: ist gleich oder verschieden bei jedem Auftreten ein aromatisches oder heteroaromatisches Ringsystem mit 5 bis 30 aromatischen Ringatomen, welches durch einen oder mehrere Reste R¹ substituiert sein kann;
   - Ar²: ist gleich oder verschieden bei jedem Auftreten eine bivalente Arylen- oder Heteroarylengruppe mit 5 bis 20 aromatischen Ringatomen, welche durch einen oder mehrere Reste R¹ substituiert sein kann;
   - R¹: ist gleich oder verschieden bei jedem Auftreten H, F, Cl, Br, I, CN, Si(R³)₃, N(R³)₂, B(OR³)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch -R³C=CR³-, -C=C-, Si(R³)₂, Ge(R³)₂, Sₙ(R³)₂, C=O, C=S, C=Se, C=NR³, -O-, -S-, -N(R³)- oder -CONR³- ersetzt sein können und wobei ein oder mehrere H-Atome durch F, Cl, Br, I, CN oder NO₂ ersetzt sein können, oder eine Aryl- oder Heteroarylgruppe mit 5 bis 30 aromatischen Ringatomen, die mit einem oder mehreren Resten R³ substituiert sein kann, oder eine Aryloxy- oder Heteroaryloxygruppe mit 5 bis 24 aromatischen Ringatomen, die durch einen oder mehrere Reste R³ substituiert sein kann, oder eine Kombination aus zwei, drei, vier oder fünf dieser Systeme; dabei können auch zwei oder mehrere Substituenten R¹ miteinander ein mono- oder polycyclisches, aliphatisches oder aromatisches Ringsystem bilden;
   - R³: ist bei jedem Auftreten gleich oder verschieden H oder ein aliphatischer oder aromatischer Kohlenwasserstoffrest mit 1 bis 20 C-Atomen; dabei können auch zwei oder mehrere Reste R³ miteinander ein mono- oder polycyclisches, aliphatisches oder aromatisches Ringsystem bilden;
   und
b) mindestens eine Verbindung der Formel (2) oder Formel (3), 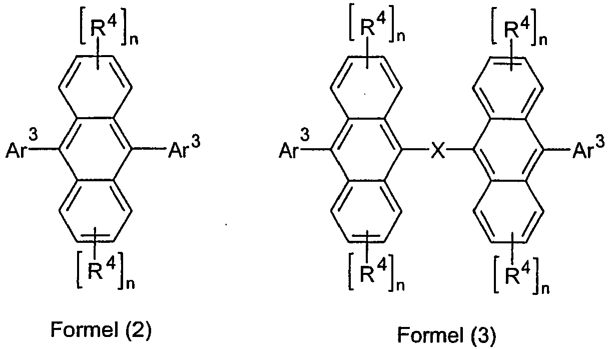
wobei für die verwendeten Symbole gilt
- Ar³: ist gleich oder verschieden bei jedem Auftreten ein aromatisches oder heteroaromatisches Ringsystem mit 5 bis 30 aromatischen Ringatomen, welches durch einen oder mehrere Reste R² substituiert sein kann;
- X: ist bei jedem Auftreten gleich oder verschieden eine bivalente Gruppe enthaltend 1 bis 40 C-Atome oder -O-, -S-, -NH- oder eine Einfachbindung;
- R², R⁴: ist gleich oder verschieden bei jedem Auftreten H, F, Cl, Br, I, CN, N(R³)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch -R³C=CR³-, -C≡C-, Si(R³)₂, Ge(R³)₂, Sn(R³)₂, C=O, C=S, C=Se, C=NR³, -O-, -S-, -N(R³)- oder -CONR³- ersetzt sein können und wobei ein oder mehrere H-Atome durch F, Cl, Br, I, CN oder NO₂ ersetzt sein können, oder eine Aryl- oder Heteroarylgruppe mit 5 bis 30 aromatischen Ringatomen, die mit einem oder mehreren Resten R³ substituiert sein kann, oder eine Aryloxy- oder Heteroaryloxygruppe mit 5 bis 24 aromatischen Ringatomen, die durch einen oder mehrere Reste R³ substituiert sein kann, oder eine Kombination aus zwei, drei, vier oder fünf dieser Systeme; dabei können auch zwei oder mehrere benachbarte Substituenten R⁴ miteinander ein mono- oder polycyclisches, aliphatisches Ringsystem bilden;
- R³: hat dieselbe Bedeutung, wie oben beschrieben;
- n: ist gleich oder verschieden bei jedem Auftreten 0, 1, 2, 3 oder 4.

Bevorzugt handelt es sich bei der organischen Schicht um eine Schicht, die zur Emission von Licht befähigt ist, insbesondere um eine emittierende Schicht.

Unter einer cyclischen Alkylgruppe im Sinne dieser Erfindung werden sowohl monocyclische wie auch bi- und polycyclische Alkylgruppen verstanden.

Unter einer Arylgruppe bzw. einer Heteroarylgruppe im Sinne dieser Erfindung wird eine aromatische Gruppe bzw. heteroaromatische Gruppe mit einem gemeinsamen aromatischen π-Elektronensystem verstanden. Dies kann im Sinne dieser Erfindung ein einfacher Homo- oder Heterocyclus sein, beispielsweise Benzol, Pyridin, Thiophen, etc., oder es kann ein kondensiertes aromatisches Ringsystem sein, in dem mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches π-Elektronensystem aufweisen. Diese Aryl- oder Heteroarylgruppen können substituiert oder unsubstituiert sein; ebenso können gegebenenfalls vorhandene Substituenten weitere Ringsysteme bilden. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische Elektronensysteme handelt.

Ein aromatisches Ringsystem im Sinne dieser Erfindung enthält 6 bis 30 C-Atome im Ringsystem. Ein heteroaromatisches Ringsystem im Sinne dieser Erfindung enthält 2 bis 30 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (weniger als 10 % der von H verschiedenen Atome, bevorzugt weniger als 5 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, etc. als aromatische Ringsysteme im Sinne dieser Erfindung verstanden werden. Dabei kann ein Teil des aromatischen oder heteroaromatischen Ringsystems auch eine kondensierte Gruppe sein.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 1 bis 30 aromatischen Ringatomen, welches noch jeweils mit den oben genannten Resten R¹ bzw. R² substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Tetracen, Pentacen, Benzpyren, Biphenyl, Biphenylen, Terphenyl, Terphenylen, Fluoren, Spirobifluoren, Truxen, Isotruxen, Dihydrophenanthren, Dihydropyren, Tetrahydropyren, cis- oder trans-Indenofluoren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Im Folgenden werden bevorzugte Ausführungsformen für Verbindungen der Formel (1) beschrieben.

Bevorzugt sind Verbindungen gemäß Formel (1), in denen das Symbol Ar², gleich oder verschieden bei jedem Auftreten, für eine bivalente Arylen- oder Heteroarylengruppe, insbesondere eine Arylengruppe, mit 6 bis 14 C-Atomen steht, welche durch einen oder mehrere Reste R¹ substituiert sein kann, ganz besonders bevorzugt für eine Phenylen- oder Naphthylengruppe, die jeweils durch einen oder mehrere Reste R¹ substituiert sein kann, insbesondere für eine Phenylengruppe, die durch einen oder mehrere Reste R¹ substituiert sein kann. Bevorzugt sind also Verbindungen der folgenden Formel (1a), wobei die Phenylengruppen noch durch einen oder mehrere Reste R¹ substituiert sein können; besonders bevorzugt sind die Phenylengruppen unsubstituiert:

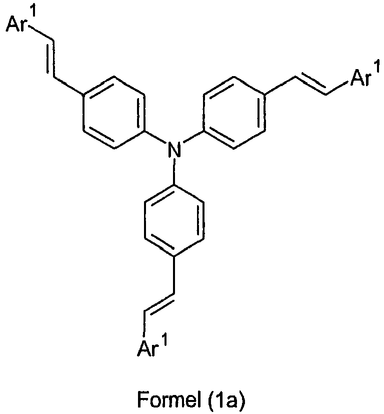

Bevorzugt sind Verbindungen gemäß Formel (1) bzw. Formel (1a), in denen das Symbol Ar¹, gleich oder verschieden bei jedem Auftreten, für eine Aryl- oder Heteroarylgruppe, insbesondere eine Arylgruppe, mit 6 bis 14 C-Atomen steht, welche durch einen oder mehrere Reste R¹ substituiert sein kann, ganz besonders bevorzugt für eine Phenyl-, 1-Naphthyl- oder 2-Naphthylgruppe, die jeweils durch einen oder mehrere Reste R¹ substituiert sein kann, insbesondere für eine Phenylgruppe, die durch einen oder mehrere Reste R¹ substituiert sein kann. Die Reste R¹ an der Gruppe Ar¹ sind bevorzugt in para-Position zur Doppelbindung gebunden.

Bevorzugte Reste R¹ sind ausgewählt aus der Gruppe bestehend aus H, F, Si(R³)₃, B(OR³)₂, geradkettigen Alkyl- oder Alkoxygruppen mit 1 bis 6 C-Atomen oder verzweigten oder cyclischen Alkyl- oder Alkoxygruppen mit 3 bis 10 C-Atomen, wobei jeweils ein oder mehrere CH₂-Gruppen durch -R³C=CR³-, Si(R³)₂, -O-, -S- oder -N(R³)- ersetzt sein können und wobei jeweils ein oder mehrere H-Atome durch F ersetzt sein können, oder monovalenten Aryl- oder Heteroarylgruppen mit 5 bis 14 aromatischen Ringatomen, oder eine Kombination aus zwei oder drei dieser Systeme. Besonders bevorzugte Reste R¹ sind ausgewählt aus der Gruppe bestehend aus H, F, Si(R³)₃, B(OR³)₂, geradkettigen Alkylgruppen mit 1 bis 4 C-Atomen, verzweigten Alkylgruppen mit 3 bis 5 C-Atomen oder cyclischen Alkylgruppen mit 5 bis 10 C-Atomen, wobei jeweils ein oder mehrere H-Atome durch F ersetzt sein können, oder monovalenten Aryl- oder Heteroarylgruppen mit 6 bis 10 aromatischen Ringatomen oder eine Kombination aus zwei dieser Systeme. Ganz besonders bevorzugte Reste R¹ sind ausgewählt aus H, F, Methyl, CF₃, Ethyl, iso-Propyl, tert-Butyl, Adamantyl, Methoxy, Trifluormethoxy, Phenyl, ortho-Tolyl, meta-Tolyl, para-Tolyl, para-Fluorphenyl, Si(Me)₃, Si(Me)₂(t-Bu), SiMe(t-Bu)₂, Si(i-Pr)₃, Boronsäureglycolester und Boronsäurepinacolester.

Ganz besonders bevorzugt sind Verbindungen gemäß Formel (1) bzw. Formel (1 a), in denen die Symbole Ar¹ für Phenylgruppen stehen, die in para-Position mit den oben genannten Resten R¹ substituiert sind.

Bevorzugt sind weiterhin Verbindungen gemäß Formel (1) bzw. Formel (1 a), in denen die Symbole Ar¹ alle gleich gewählt sind. Besonders bevorzugt sind Verbindungen gemäß Formel (1) bzw. Formel (1a), in denen die Gruppen Ar¹ alle gleich gewählt sind und alle mit den gleichen Substituenten R¹ in den gleichen Positionen substituiert sind, also Verbindungen, die eine dreizählige Symmetrieachse aufweisen.

Beispiele für bevorzugte Verbindungen gemäß Formel (1) sind die im Folgenden abgebildeten Verbindungen (S1) bis (S87).

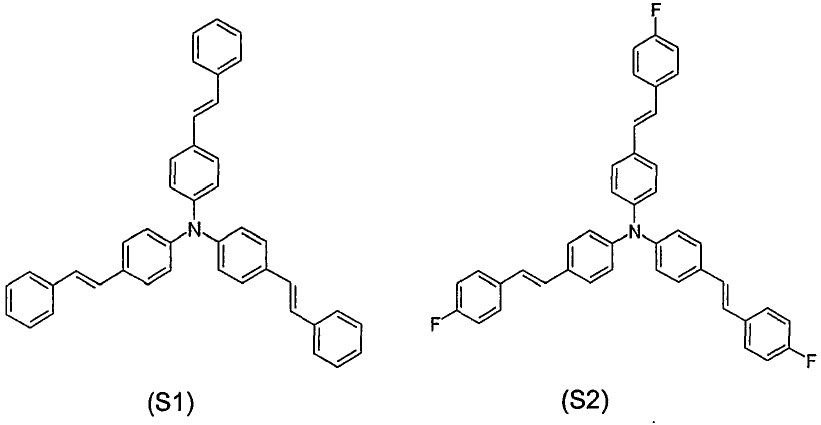

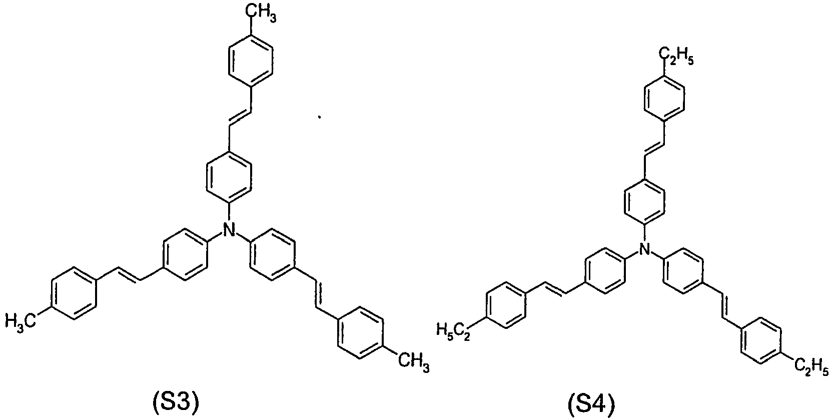

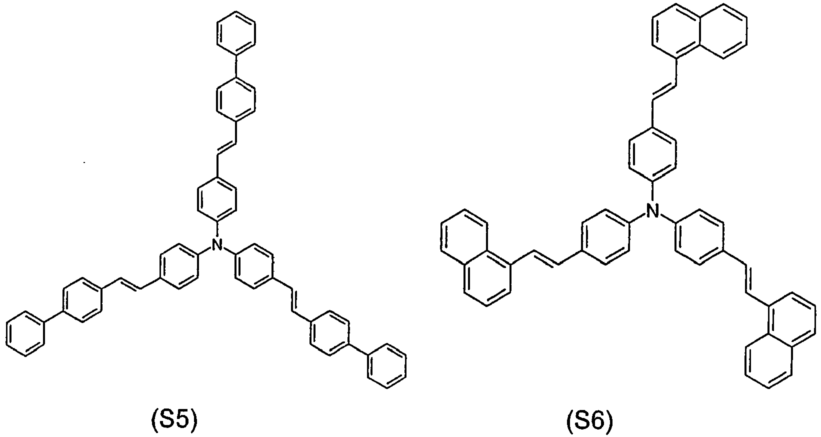

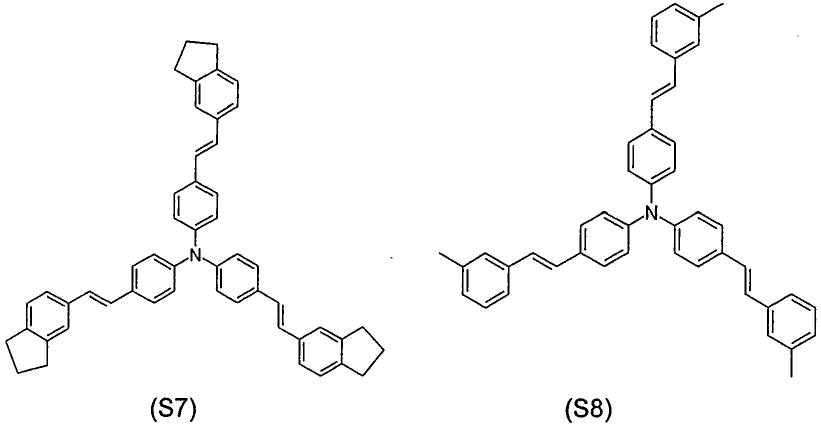

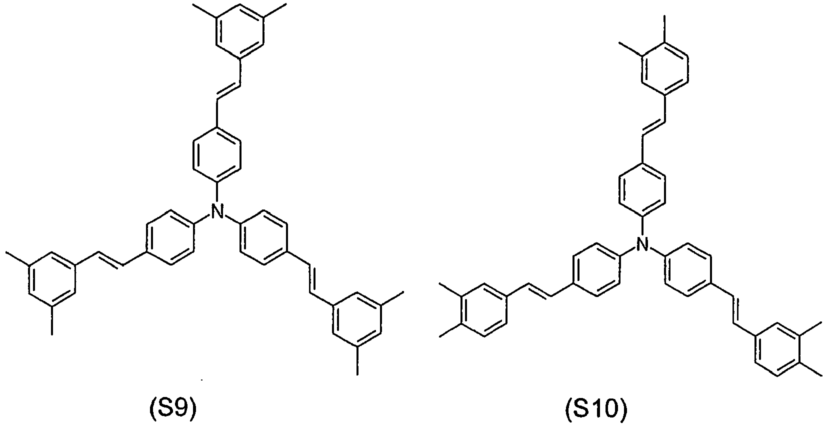

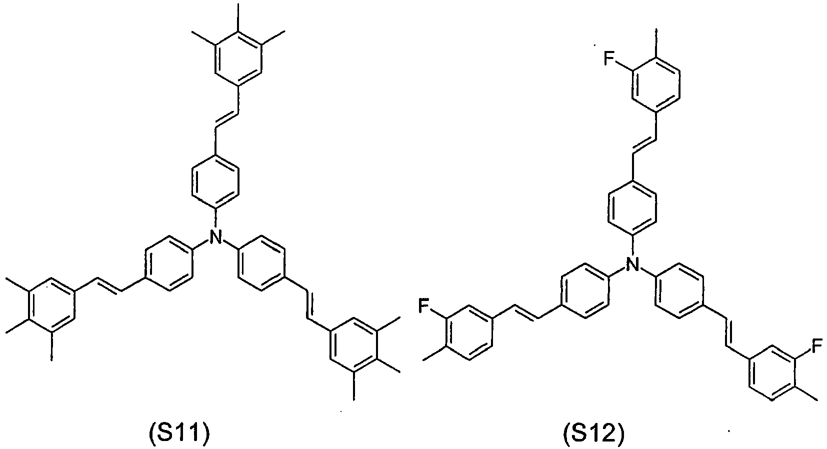

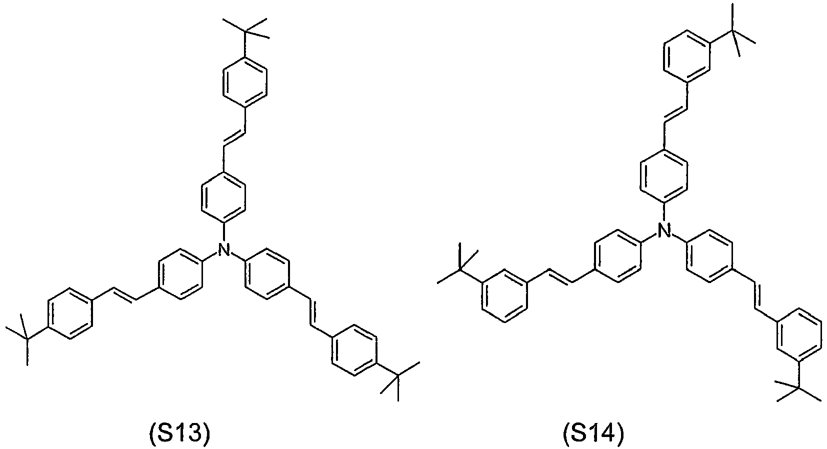

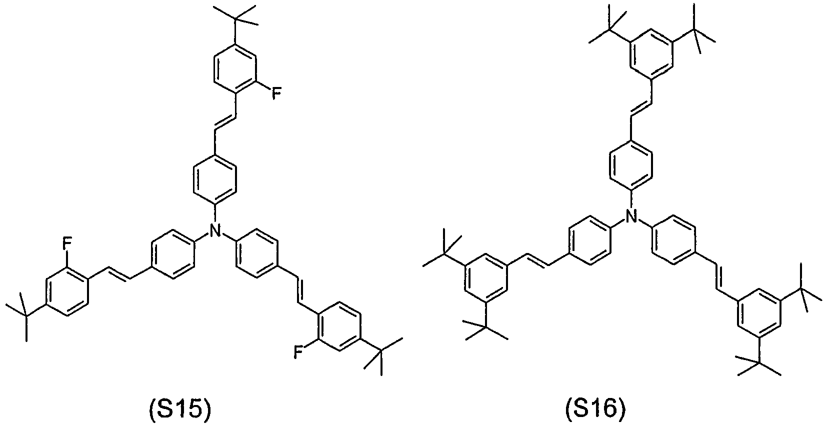

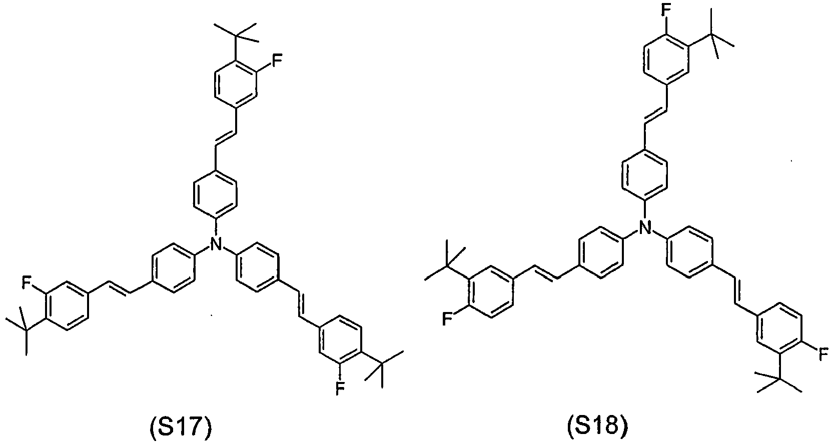

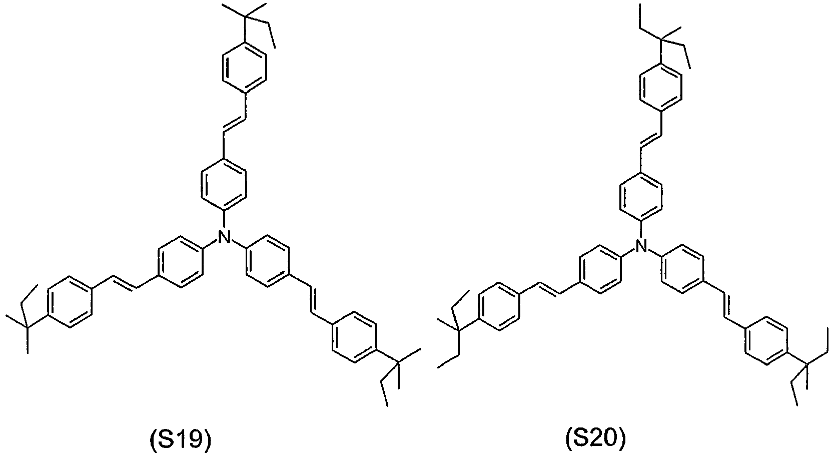

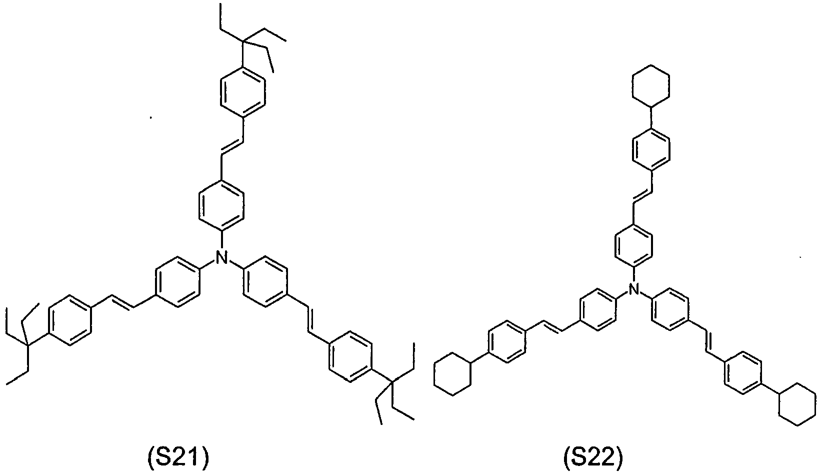

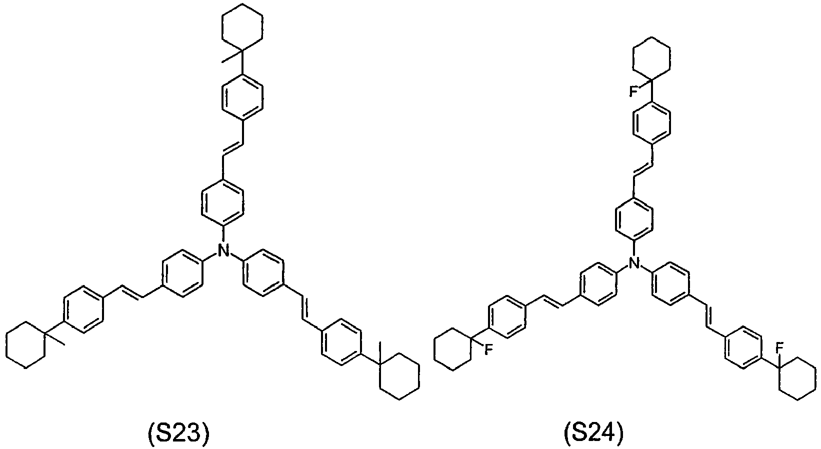

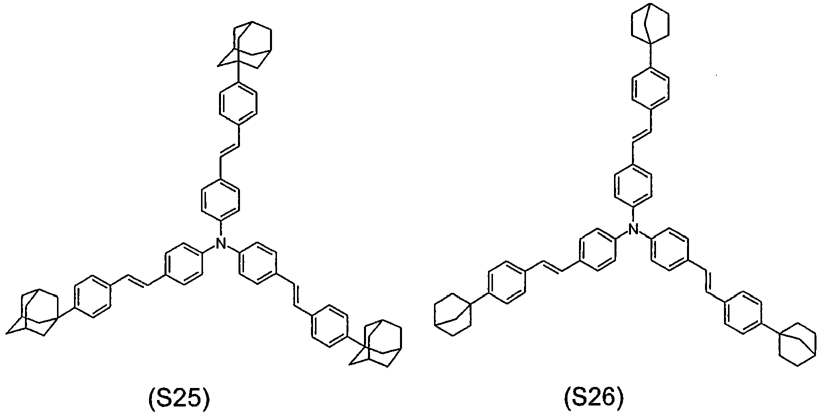

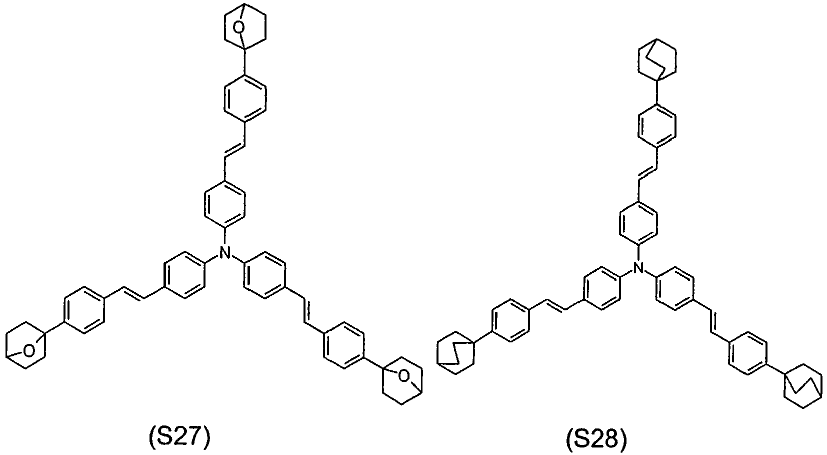

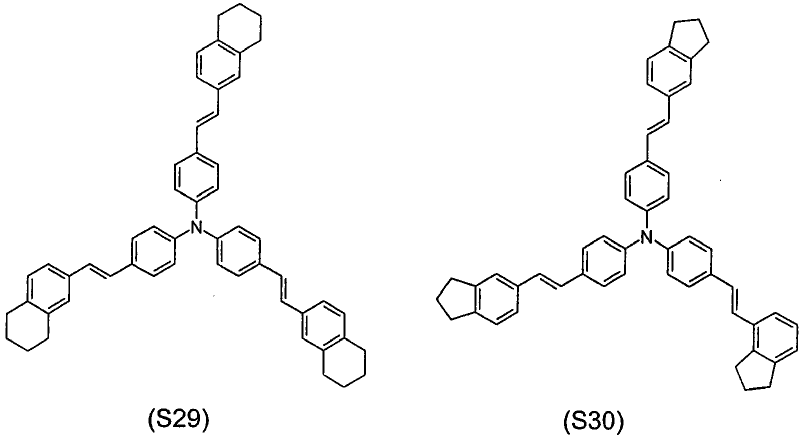

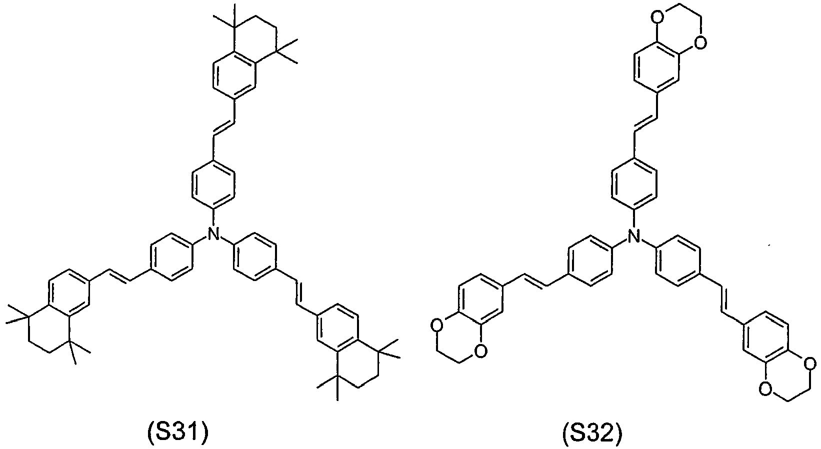

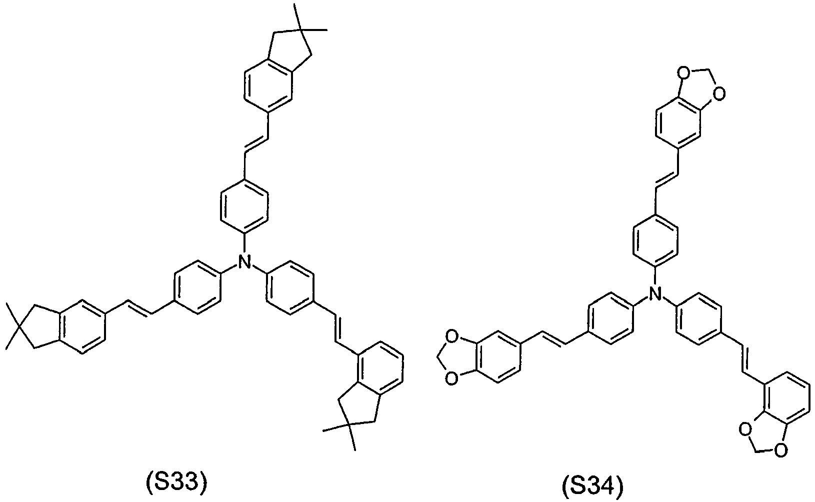

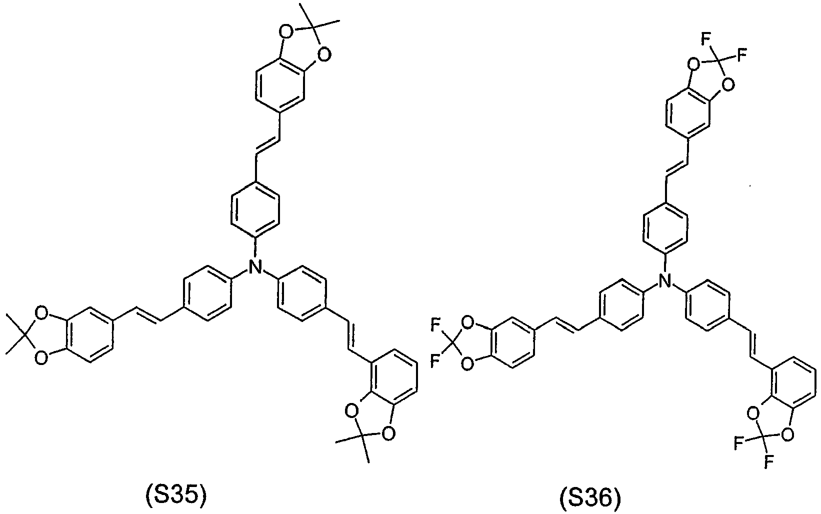

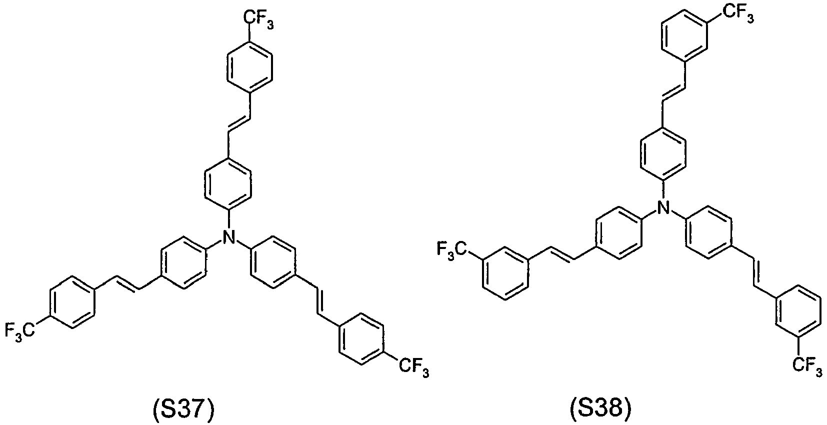

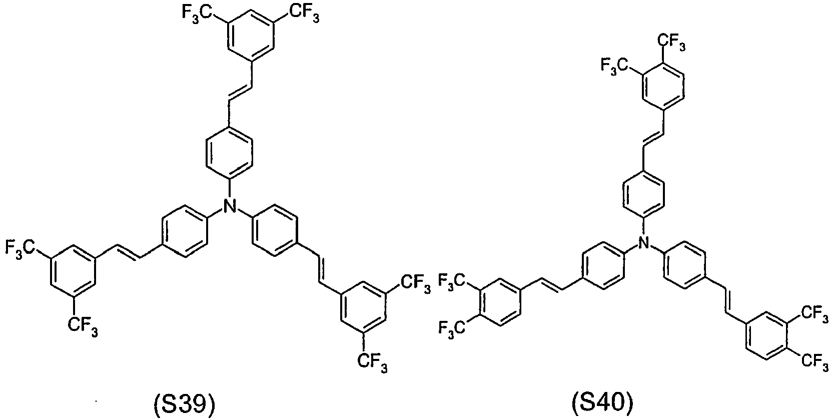

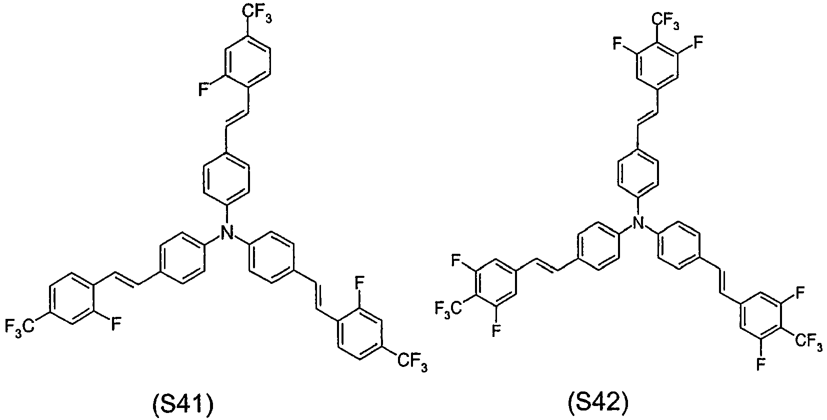

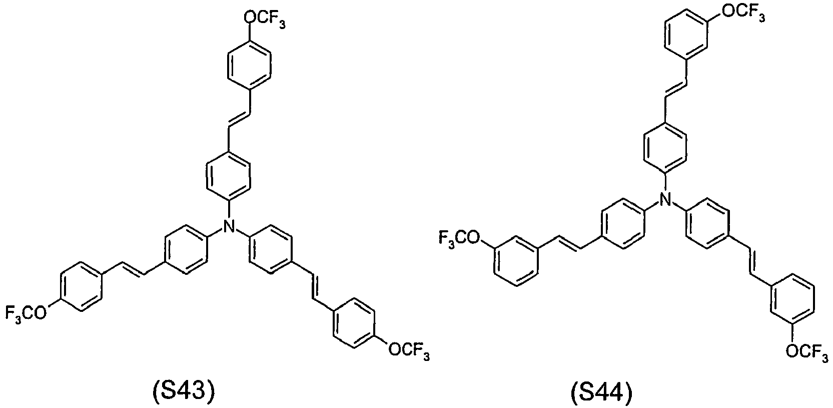

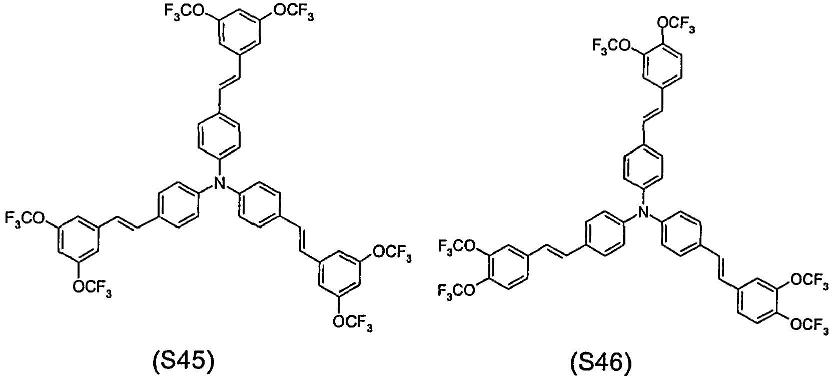

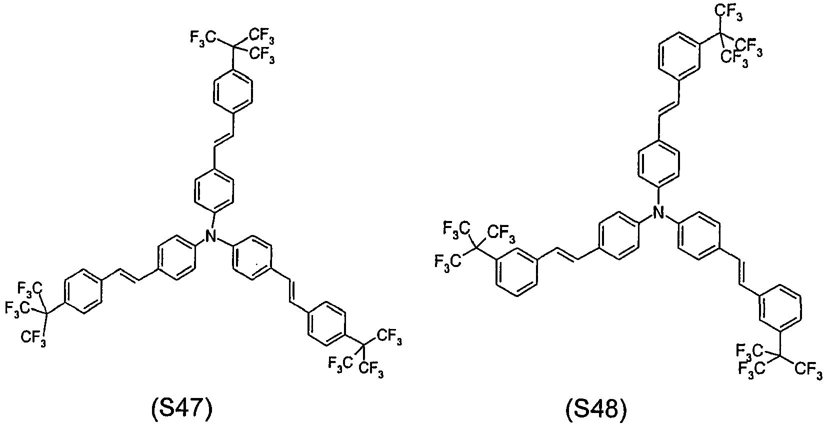

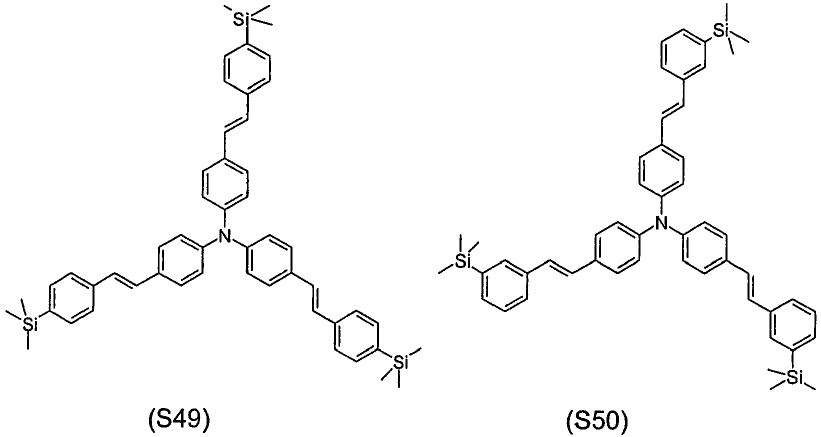

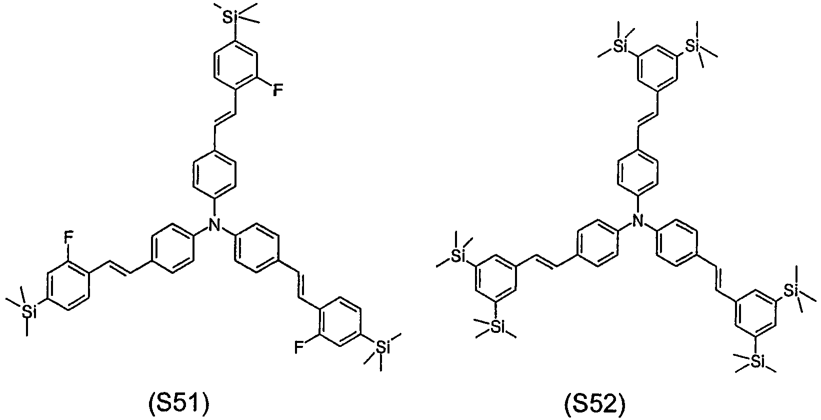

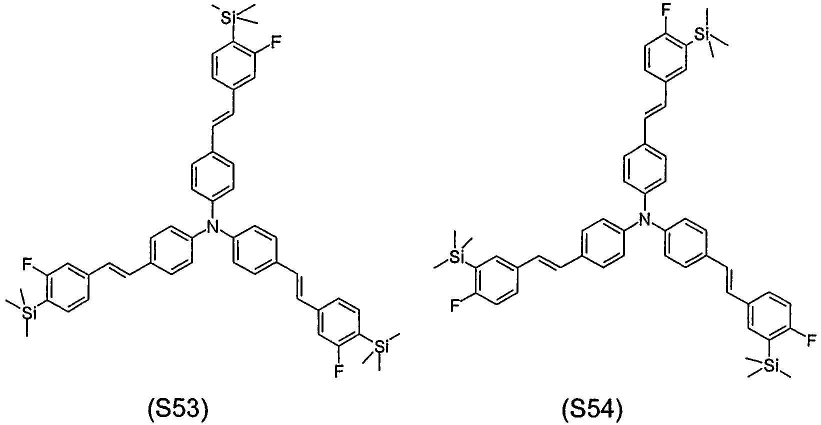

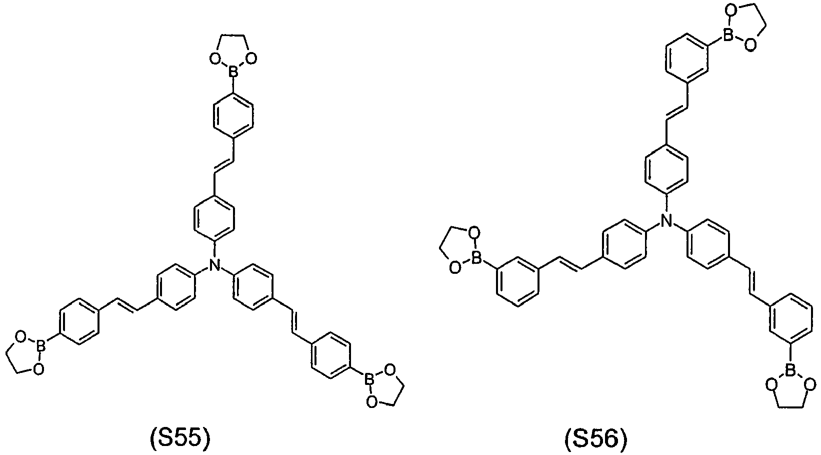

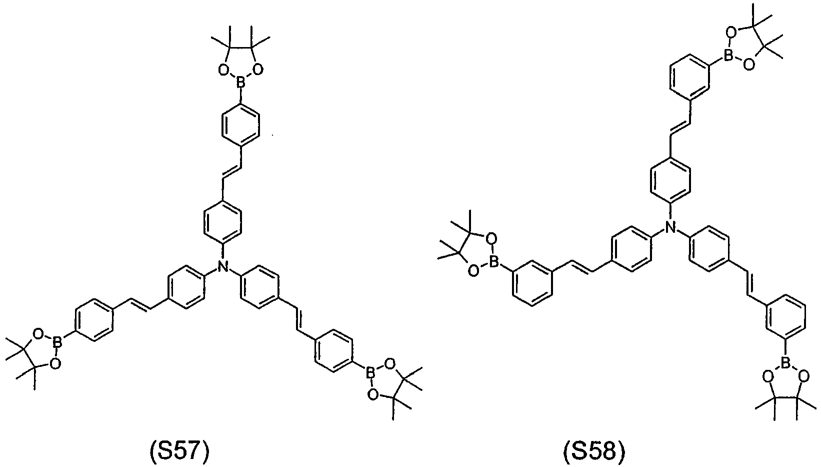

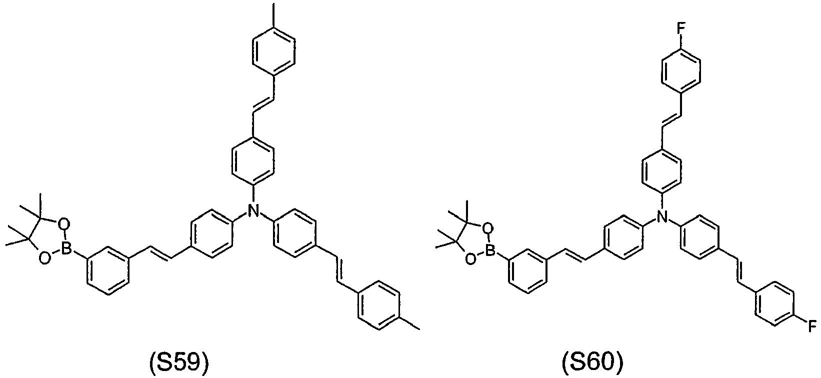

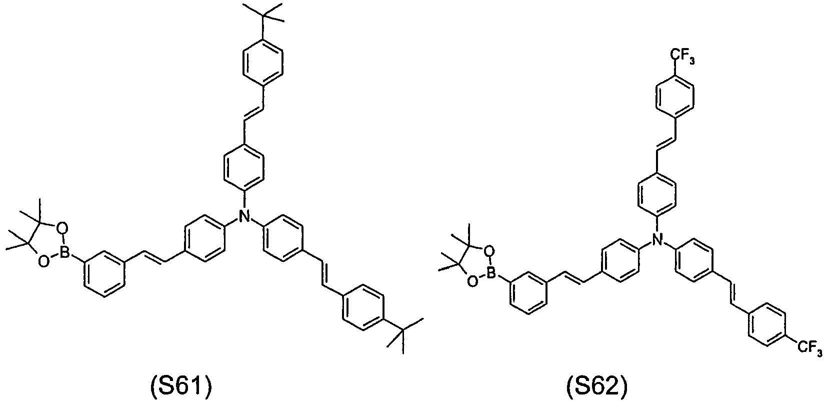

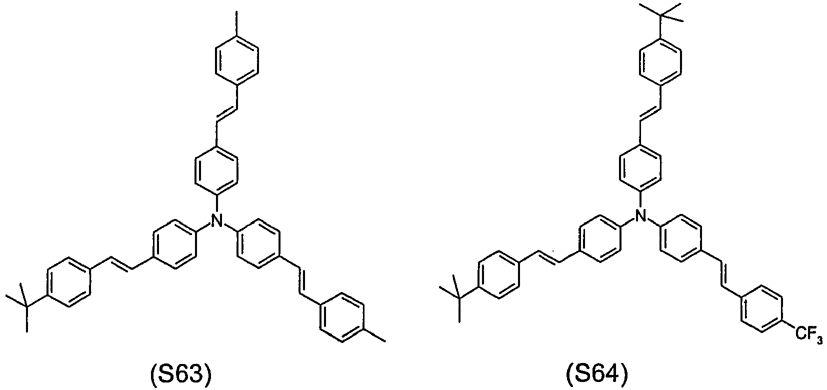

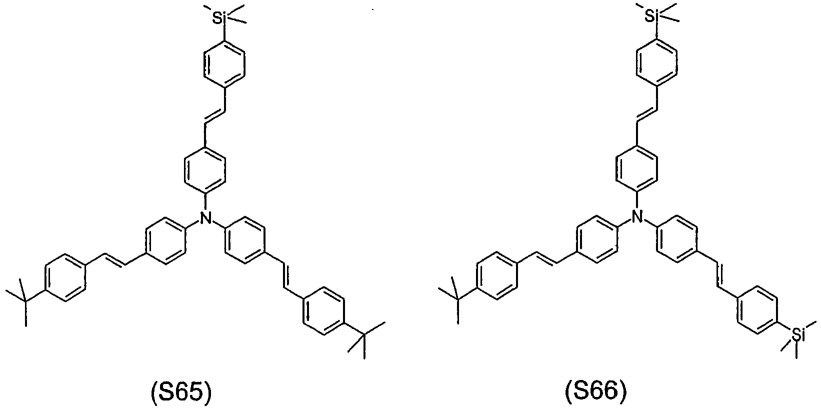

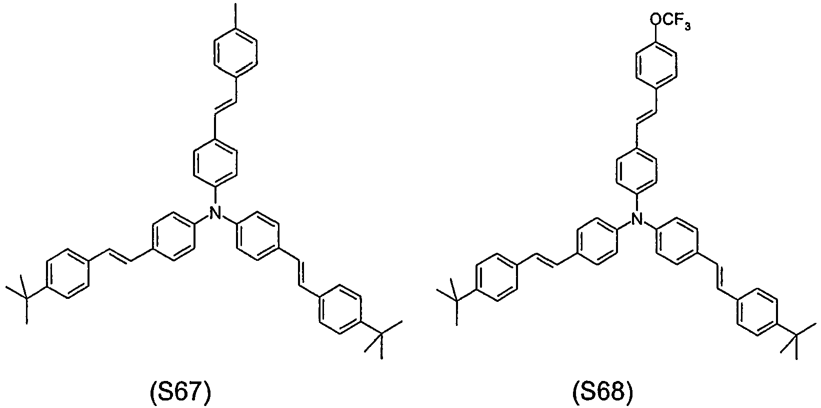

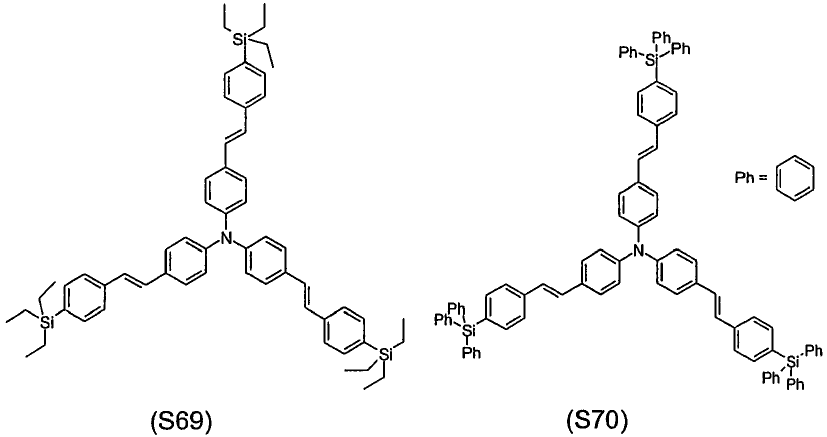

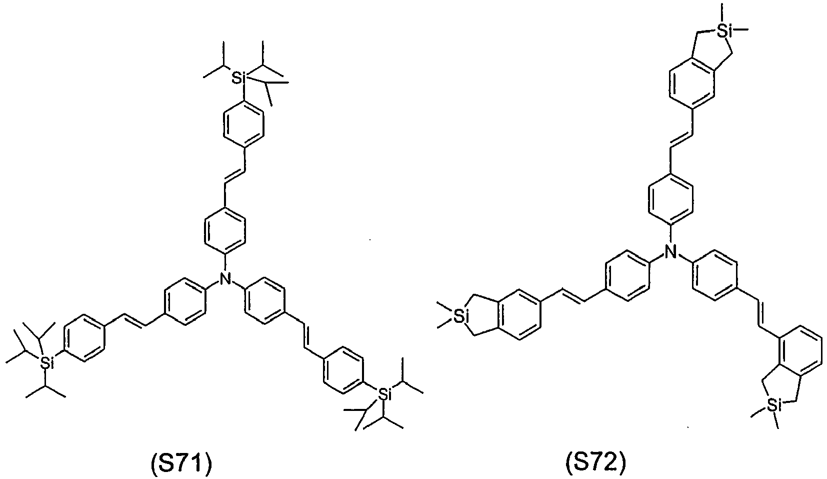

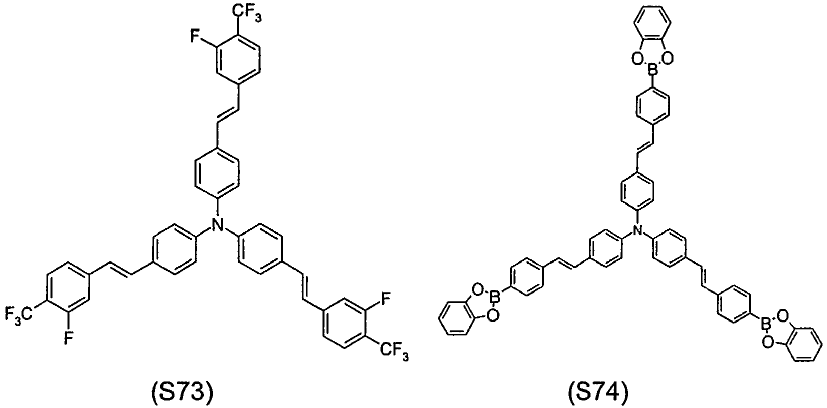

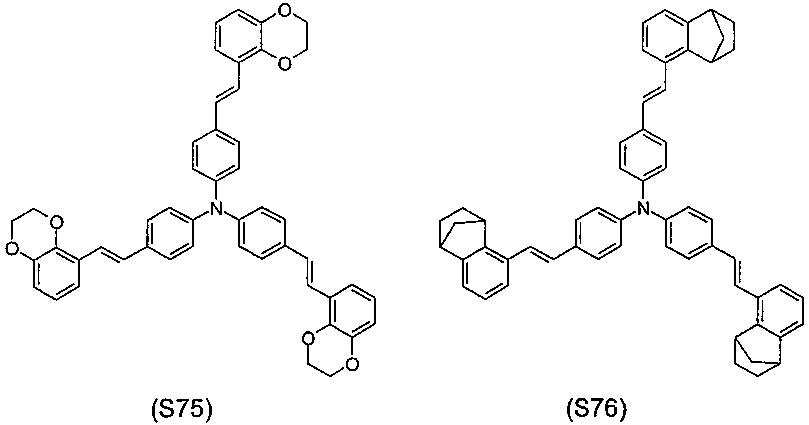

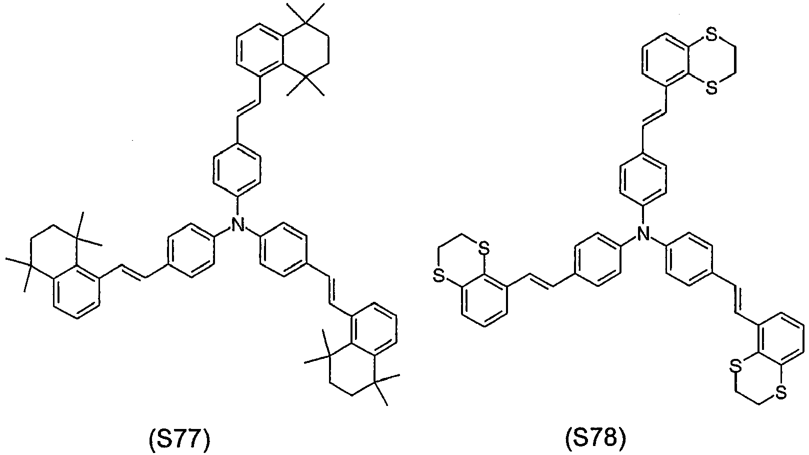

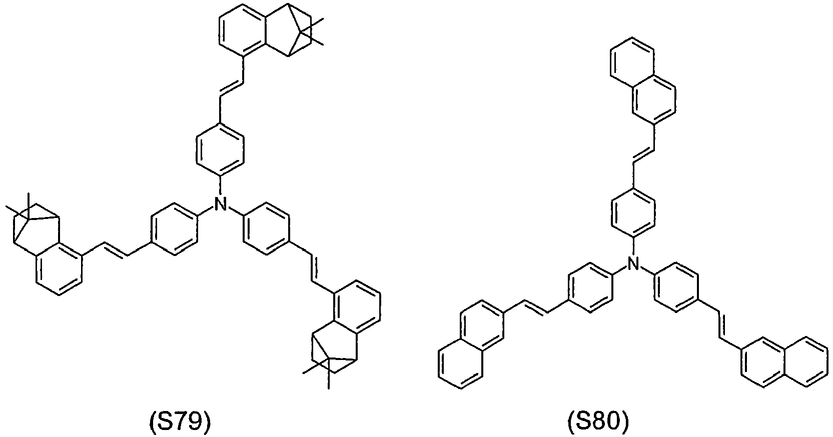

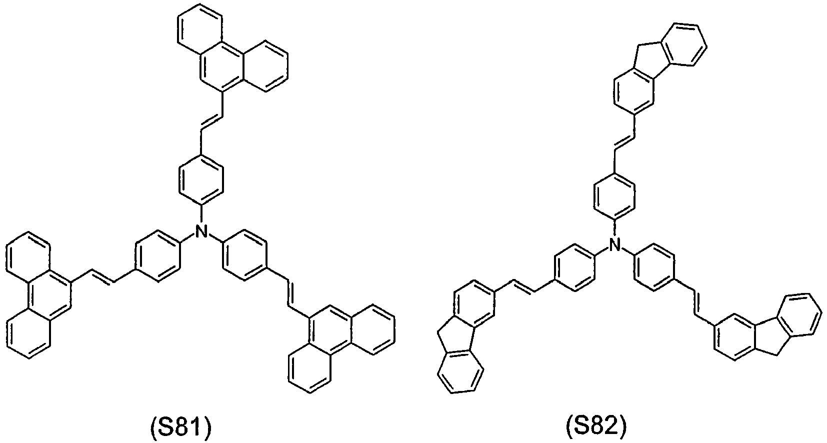

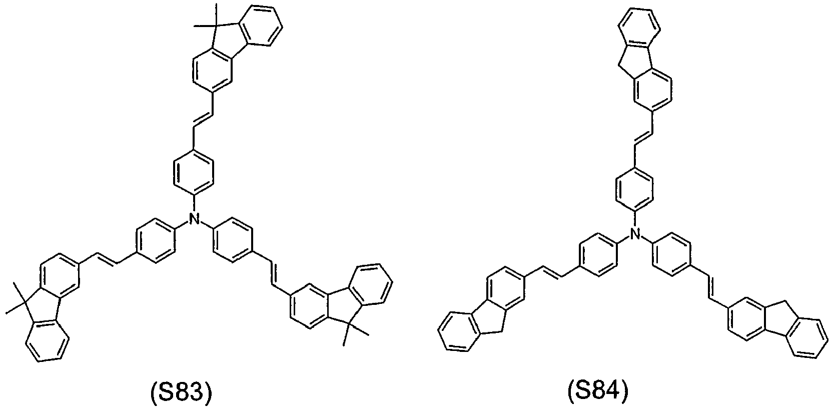

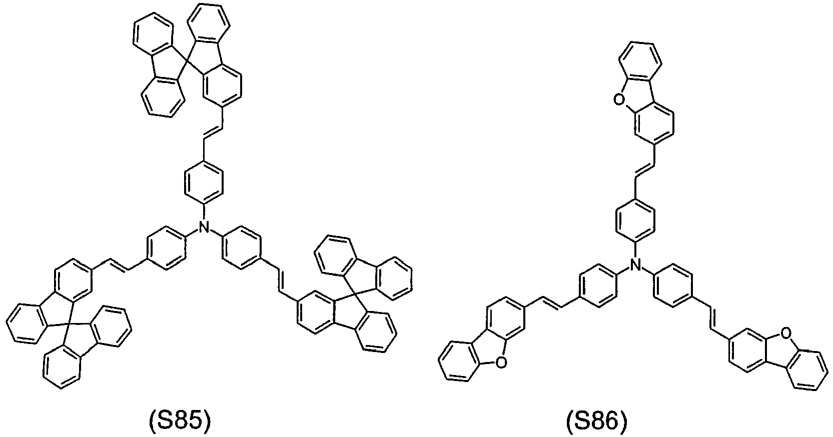

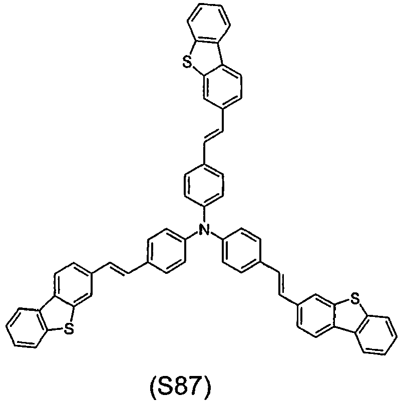

Im Folgenden werden bevorzugte Ausführungsformen für Verbindungen der Formel (2) und Formel (3) beschrieben.

Bevorzugt sind Verbindungen der Formel (2) und Formel (3), in denen das Symbol Ar³, gleich oder verschieden bei jedem Auftreten, für ein aromatisches oder heteroaromatisches Ringsystem mit 9 bis 25 aromatischen Ringatomen steht, welches durch einen oder mehrere Reste R² substituiert sein kann. Besonders bevorzugt sind Verbindungen der Formel (2) und Formel (3), in denen das Symbol Ar³, gleich oder verschieden bei jedem Auftreten, für eine kondensierte Aryl- oder Heteroarylgruppe mit 10 bis 16 aromatischen Ringatomen oder für eine aromatische, gegebenenfalls überbrückte, Biarylgruppe steht, die jeweils mit einem oder mehreren Resten R² substituiert sein können. Ganz besonders bevorzugt sind Verbindungen der Formel (2), in denen das Symbol Ar³, gleich oder verschieden bei jedem Auftreten, für eine 1-Naphthyl-, 2-Naphthyl-, 9-Anthryl-, 2-Phenanthrenyl-, 9-Phenanthrenyl-, Chinolinyl-, Isochinolinyl-, ortho-Biphenyl-, 2-Fluorenyl- oder 2-Spirobifluorenylgruppe steht, die jeweils mit einem oder mehreren Resten R² substituiert sein kann, insbesondere 1-Naphthyl, welches mit einem oder mehreren Resten R² substituiert sein kann.

Bevorzugt sind weiterhin Verbindungen gemäß Formel (2) und Formel (3), in denen das Symbol R², gleich oder verschieden bei jedem Auftreten, für H, F, eine geradkettige Alkyl- oder Alkoxygruppe mit 1 bis 6 C-Atomen oder eine verzweigte oder cyclische Alkyl- oder Alkoxygruppe mit 3 bis 10 C-Atomen, wobei jeweils ein oder mehrere CH₂-Gruppen durch Si(R³)₂, -R³C=CR³-, -O-, -S- oder -N(R³)- ersetzt sein können und wobei jeweils ein oder mehrere H-Atome durch F ersetzt sein können, oder eine Aryl- oder Heteroarylgruppe mit 5 bis 14 aromatischen Ringatomen, oder eine Kombination aus zwei oder drei dieser Systeme steht; dabei können auch zwei oder mehrere Reste R² miteinander ein mono- oder polycyclisches, aliphatisches oder aromatisches Ringsystem bilden. Besonders bevorzugte Reste R² sind ausgewählt aus der Gruppe bestehend aus H, F, geradkettigen Alkylgruppen mit 1 bis 4 C-Atomen, verzweigten Alkylgruppen mit 3 bis 5 C-Atomen oder cyclischen Alkylgruppen mit 5 bis 10 C-Atomen, wobei jeweils ein oder mehrere H-Atome durch F ersetzt sein können, oder Aryl- oder Heteroarylgruppen mit 6 bis 10 aromatischen Ringatomen oder eine Kombination aus zwei dieser Systeme; dabei können auch zwei oder mehrere benachbarte Reste R² miteinander ein mono- oder polycyclisches, aliphatisches oder aromatisches Ringsystem bilden.

Bevorzugt sind weiterhin Verbindungen gemäß Formel (3), in denen das Symbol X, gleich oder verschieden bei jedem Auftreten, eine lineare Alkylengruppe mit 1 bis 10 C-Atomen oder eine verzweigte oder cyclische Alkylen- oder Alkylidengruppe mit 3 bis 10 C-Atomen, eine bivalente aromatische Gruppe mit 6 bis 25 C-Atomen, -O-, -S- oder eine Gruppe der Formel -N(R³)- oder -P(=O)R³- oder eine Einfachbindung darstellt.

Bevorzugt sind weiterhin Verbindungen gemäß Formel (2) bzw. Formel (3), in denen das Symbol R⁴, gleich oder verschieden bei jedem Auftreten, für H, F, eine geradkettige Alkyl- oder Alkoxygruppe mit 1 bis 6 C-Atomen oder eine verzweigte oder cyclische Alkyl- oder Alkoxygruppe mit 3 bis 10 C-Atomen, wobei jeweils ein oder mehrere CH₂-Gruppen durch -R³C=CR³-, Si(R³)₂, -O-, -S- oder -N(R³)- ersetzt sein können und wobei jeweils ein oder mehrere H-Atome durch F ersetzt sein können, oder eine Aryl- oder Heteroarylgruppe mit 5 bis 14 aromatischen Ringatomen, oder eine Kombination aus zwei oder drei dieser Systeme steht; dabei können auch zwei oder mehrere Reste R⁴ miteinander ein mono- oder polycyclisches, aliphatisches Ringsystem bilden. Besonders bevorzugte Reste R⁴ sind ausgewählt aus der Gruppe bestehend aus H, F, geradkettigen Alkylgruppen mit 1 bis 4 C-Atomen, verzweigten Alkylgruppen mit 3 bis 5 C-Atomen oder cyclischen Alkylgruppen mit 5 bis 10 C-Atomen, wobei jeweils ein oder mehrere H-Atome durch F ersetzt sein können, oder Aryl- oder Heteroarylgruppen mit 6 bis 10 aromatischen Ringatomen oder einer Kombination aus zwei dieser Systeme; dabei können auch zwei oder mehrere benachbarte Reste R⁴ miteinander ein mono- oder polycyclisches, aliphatisches Ringsystem bilden.

Bevorzugt sind weiterhin Verbindungen gemäß Formel (2) bzw. Formel (3), in denen der Index n für 0, 1 oder 2 steht, besonders bevorzugt für 0 oder 1. Falls der Index n für 1 steht, ist der Substituent R⁴ bevorzugt in 2-Position und/oder in 6-Position des Anthracens gebunden.

Bevorzugt sind weiterhin Verbindungen gemäß Formel (2) bzw. Formel (3), deren Molekulargewicht zwischen 400 und 1200 g/mol liegt, besonders bevorzugt zwischen 400 und 900 g/mol.

Bevorzugt sind die Verbindungen gemäß Formel (2) bzw. Formel (3) reine Kohlenwasserstoffverbindungen.

Dabei sei betont, dass sowohl Verbindungen gemäß Formel (2) bzw. Formel (3) erfindungsgemäß sind, in denen die beiden Gruppen Ar³ gleich gewählt sind, wie auch Verbindungen gemäß Formel (2) bzw. Formel (3), in denen die beiden Gruppen Ar³ unterschiedlich sind.

Bevorzugt sind weiterhin Verbindungen gemäß Formel (2) bzw. Formel (3), die gehinderte Rotation um die Ar³-Anthracen-Bindung bzw. gegebenenfalls auch um die Anthracen-X-Bindung aufweisen und die dadurch zur Bildung von Atropisomeren befähigt sind. Unter "gehinderter Rotation" im Sinne dieser Erfindung wird eine Rotationsbarriere von mindestens 80 kJ/mol, bevorzugt mindestens 100 kJ/mol, besonders bevorzugt mindestens 120 kJ/mol bei Raumtemperatur verstanden. Diese Rotationsbarriere lässt sich experimentell durch temperaturabhängige NMR-Messungen bestimmen. Derartige Verbindungen gemäß Formel (2) bzw. Formel (3) sind beispielsweise in WO 06/048268 beschrieben. Wenn die Verbindung der Formel (2) bzw. Formel (3) Atropisomerie um eine oder um mehrere Bindungen zeigt, wie oben beschrieben, so sind jeweils auch OLEDs enthaltend die entsprechenden isolierten oder angereicherten Atropisomere Gegenstand der Erfindung. Dies bezieht sich sowohl auf Enantiomere wie auch auf Diastereomere. Durch die Wahl geeigneter Atropisomere lassen sich beispielsweise die Löslichkeit der Verbindung und die elektrooptischen Eigenschaften beeinflussen.

Beispiele für bevorzugte Verbindungen gemäß Formel (2) und Formel (3) sind die im Folgenden abgebildeten Verbindungen (A1) bis (A64).

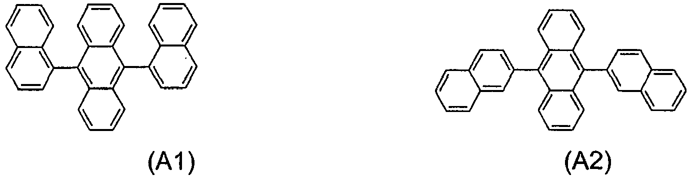

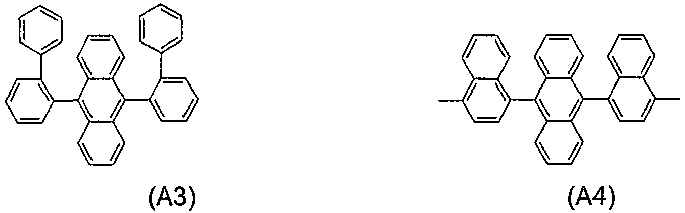

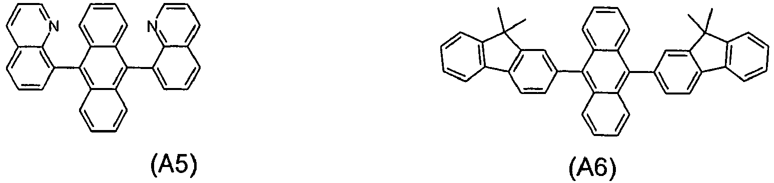

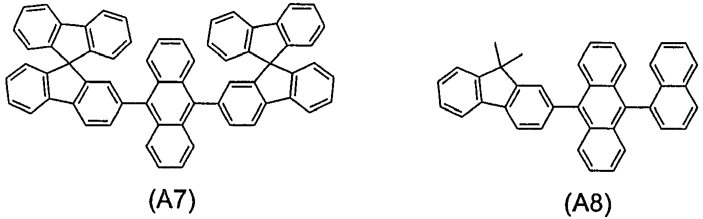

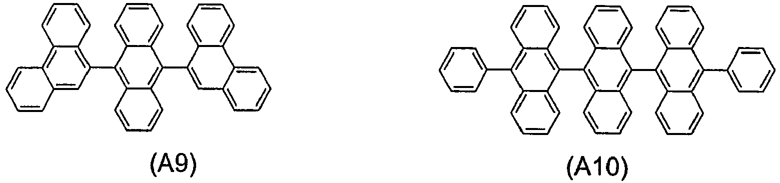

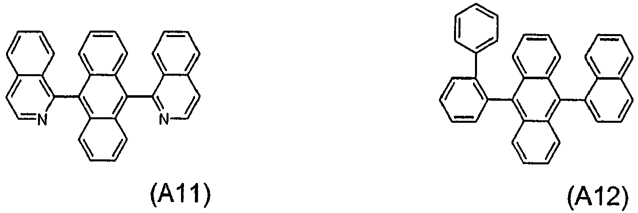

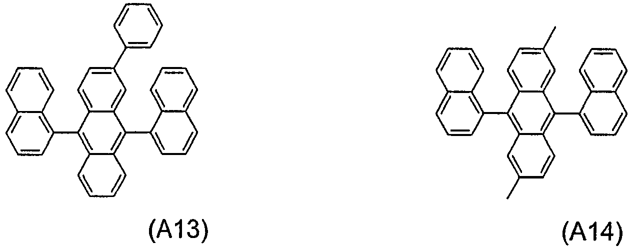

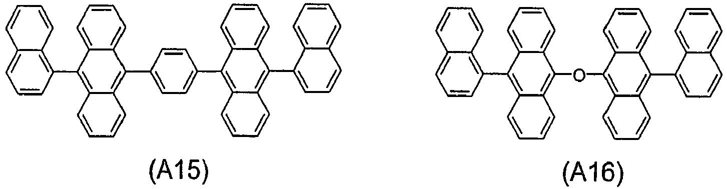

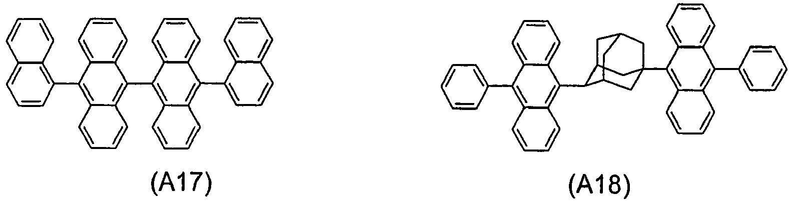

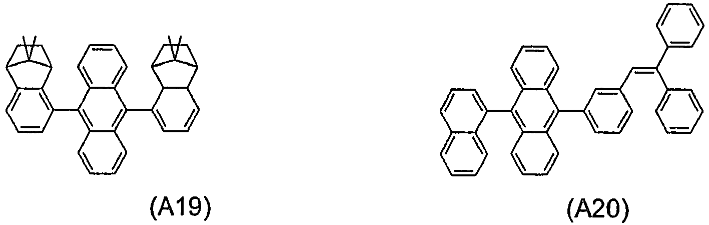

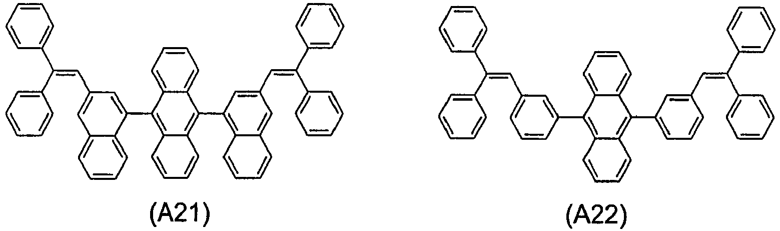

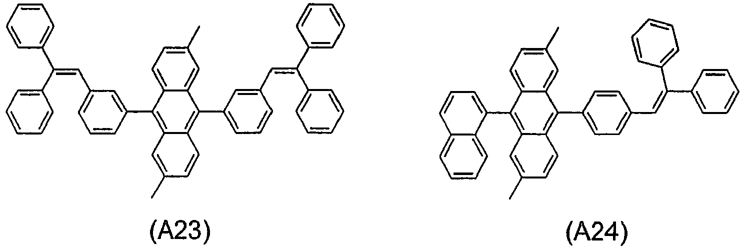

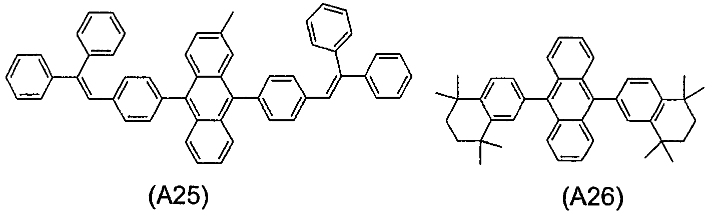

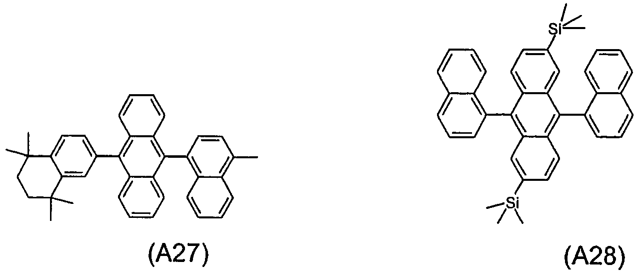

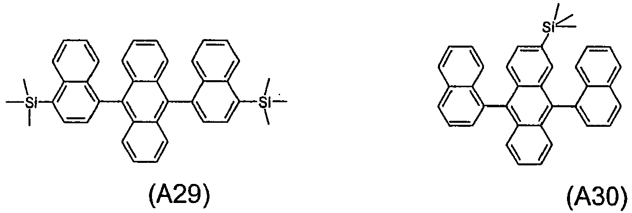

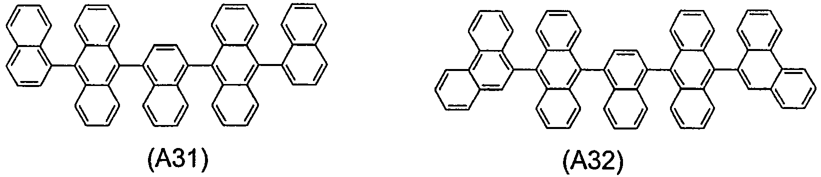

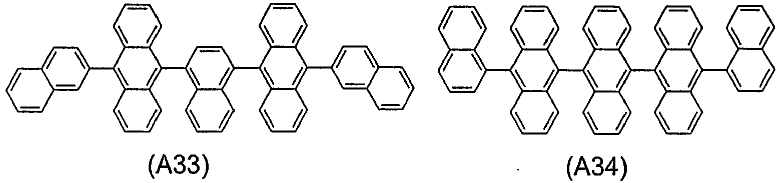

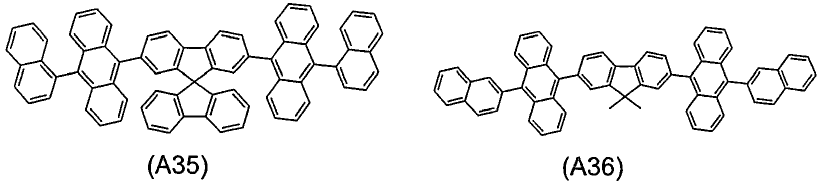

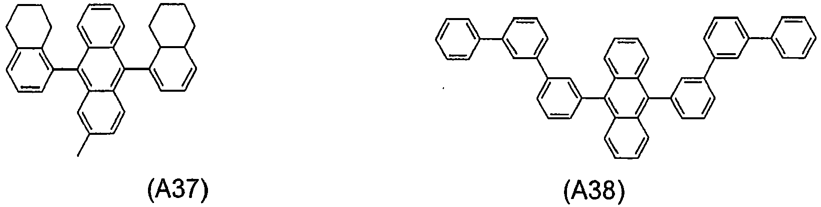

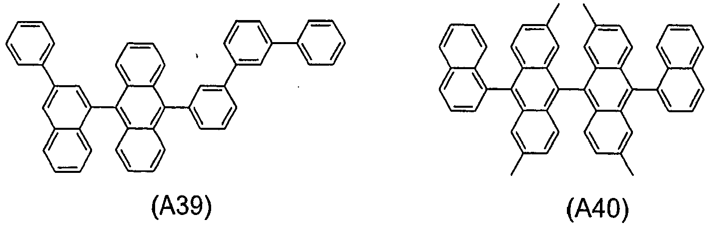

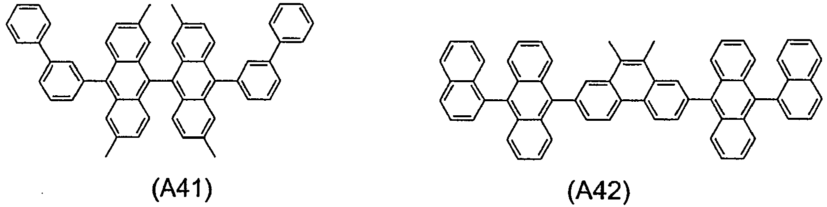

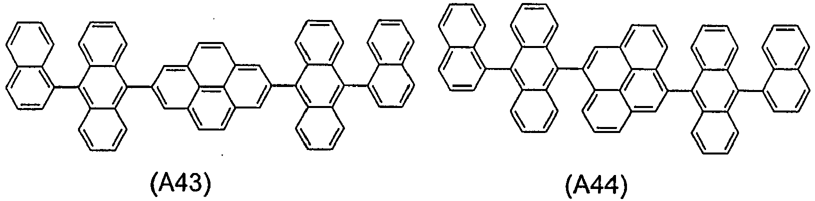

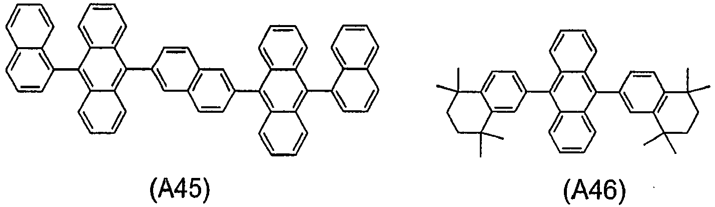

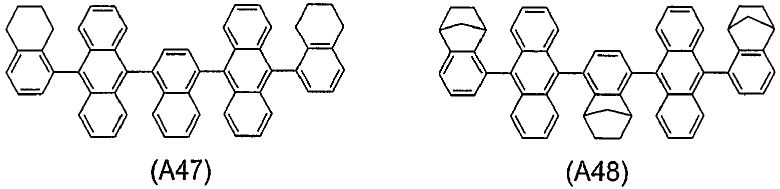

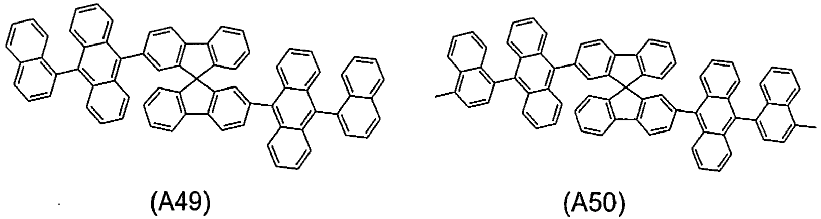

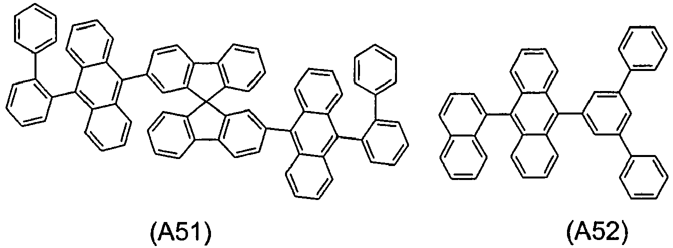

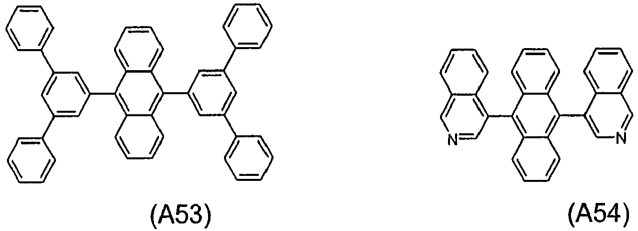

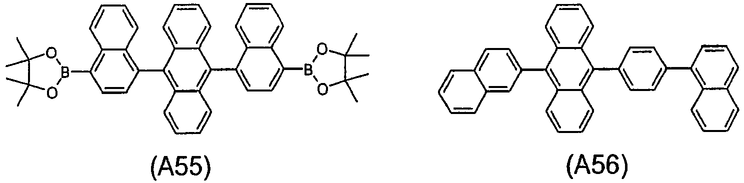

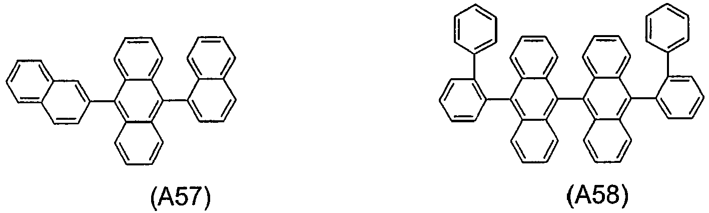

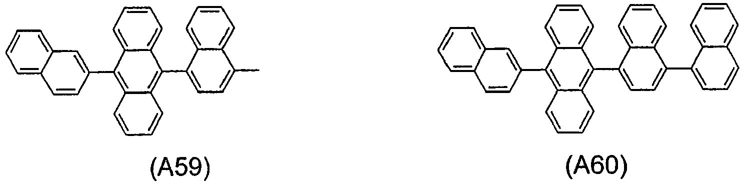

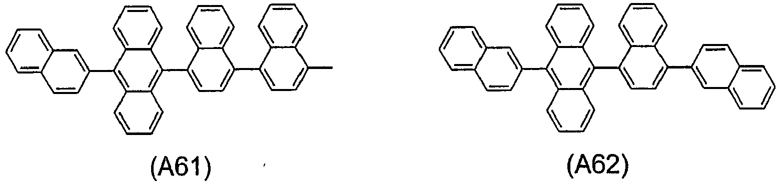

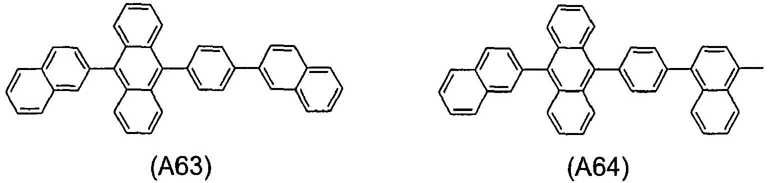

Der Anteil der Verbindung gemäß Formel (1) bzw. Formel (1 a) in der Mischung beträgt zwischen 0.1 und 99.0 Gew.-%, bevorzugt zwischen 0.5 und 50.0 Gew.-%, besonders bevorzugt zwischen 1.0 und 20.0 Gew.-%, insbesondere zwischen 1.0 und 10.0 Gew.-%. Entsprechend beträgt der Anteil der Verbindung gemäß Formel (2) bzw. Formel (3) in der Mischung zwischen 1.0 und 99.9 Gew.-%, bevorzugt zwischen 50.0 und 99.5 Gew.-%, besonders bevorzugt zwischen 80.0 und 99.0 Gew.-%, insbesondere zwischen 90.0 und 99.0 Gew.-%.

Weiterhin bevorzugt sind organische Elektrolumineszenzvorrichtungen, dadurch gekennzeichnet, dass mehrere emittierende Schichten verwendet werden, wobei mindestens eine dieser Schichten mindestens eine Verbindung gemäß Formel (1) bzw. Formel (1a) und mindestens eine Verbindung gemäß Formel (2) bzw. Formel (3) enthält. Besonders bevorzugt weisen diese Emissionsschichten insgesamt mehrere Emissionsmaxima zwischen 380 nm und 750 nm auf, so dass insgesamt weiße Emission resultiert, d. h. in der bzw. den weiteren emittierenden Schichten wird noch mindestens eine weitere emittierende Verbindung verwendet, die fluoreszieren oder phosphoreszieren kann und die gelbes, orange oder rotes Licht emittiert. Insbesondere bevorzugt sind Dreischichtsysteme, wobei mindestens eine dieser Schichten mindestens eine Verbindung gemäß Formel (1) bzw. Formel (1a) und eine Verbindung gemäß Formel (2) bzw. Formel (3) enthält und wobei die drei Schichten blaue, grüne und orange oder rote Emission zeigen (für den prinzipiellen Aufbau siehe z. B. WO 05/011013).

Außer den Verbindungen gemäß Formel (1) bzw. Formel (1a) und Formel (2) bzw. Formel (3) können in der emittierenden Schicht auch weitere Substanzen vorhanden sein, beispielsweise Loch- oder Elektronentransportmaterialien.

Außer Kathode, Anode und der emittierenden Schicht (bzw. den emittierenden Schichten) kann die organische Elektrolumineszenzvorrichtung noch weitere Schichten enthalten. Diese können beispielsweise sein: Lochinjektionsschicht, Lochtransportschicht, Elektronentransportschicht und/oder Elektroneninjektionsschicht. Die Materialien in diesen Schichten können auch dotiert sein. Es muss aber nicht notwendigerweise jede dieser Schichten vorhanden sein muss. Als Lochtransportmaterialien eignen sich beispielsweise aromatische Amine, wie sie üblicherweise gemäß dem Stand der Technik verwendet werden, welche auch p-dotiert sein können. Als Elektronentransportmaterialien eignen sich beispielsweise Metallchelatkomplexe, z. B. AlQ₃, Verbindungen auf Basis elektronenarmer Heterocyclen, z. B. Triazinderivate, oder Verbindungen enthaltend aromatische Carbonyle oder Phosphinoxide, wie z. B. beschrieben in WO 05/084081 und WO 05/084082, welche jeweils auch n-dotiert sein können. Als Elektroneninjektionsmaterialien eignen sich insbesondere Fluoride und Oxide der Alkali- und Erdalkalimetalle, beispielsweise NaF, BaF₂, CaF₂, LiF oder Li₂O

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) und Formel (2) bzw. Formel (3) nötig. Hohe Löslichkeit lässt sich entweder durch geeignete Substitution der Verbindungen oder aber auch durch die Wahl geeigneter Atropisomere erreichen.

Ein weiterer Gegenstand der Erfindung ist ein Verfahren zur Herstellung von organischen Elektrolumineszenzvorrichtungen, dadurch gekennzeichnet, dass mindestens eine Verbindung gemäß Formel (1) zusammen mit mindestens einer Verbindung gemäß Formel (2) und/oder Formel (3) durch ein Sublimationsverfahren oder aus Lösung, beispielsweise durch ein Druckverfahren, aufgebracht werden.

Weiterer Gegenstand sind Mischungen enthaltend mindestens eine Verbindung gemäß Formel (1) bzw. Formel (1a) und mindestens eine Verbindung gemäß Formel (2) bzw. Formel (3), wobei für die Mischungen die oben ausgeführten Bevorzugungen zutreffen.

Weiterer Gegenstand der Erfindung ist die Verwendung der erfindungsgemäßen Mischungen zur Herstellung organischer elektronischer Vorrichtungen, insbesondere organischer Elektrolumineszenzvorrichtungen.

Die erfindungsgemäßen organischen Elektrolumineszenzvorrichtungen folgende überraschende Vorteile gegenüber dem Stand der Technik auf:
1. Die Stabilität der Vorrichtungen wird höher im Vergleich zu Systemen gemäß dem Stand der Technik, was sich vor allem in einer deutlich höheren Lebensdauer zeigt.
2. Die Emissionsfarbe der Vorrichtungen ist deutlich tiefer blau im Vergleich zu Vorrichtungen enthaltend die üblicherweise verwendeten Systeme gemäß dem Stand der Technik. Dadurch eignen sich die erfindungsgemäßen Vorrichtungen besser für die Verwendung in hochwertigen Vollfarbdisplays.
3. Die Verbindungen lassen sich gut und ohne erhebliche Zersetzung sublimieren und aufdampfen, sind dadurch leichter zu verarbeiten und deshalb besser für die Verwendung in OLEDs geeignet als Materialien gemäß dem Stand der Technik.

Im vorliegenden Anmeldetext und auch in den im Weiteren folgenden Beispielen wird auf die Verwendung erfindungsgemäßer Mischungen in Bezug auf OLEDs und die entsprechenden Displays abgezielt.

Trotz dieser Beschränkung der Beschreibung ist es für den Fachmann ohne weiteres erfinderisches Zutun möglich, die erfindungsgemäßen Mischungen auch für weitere Verwendungen in anderen elektronischen Vorrichtungen zu benutzen, z. B. für organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), organische Photorezeptoren oder organische Laserdioden (O-Laser).

Die Verwendung der erfindungsgemäßen Mischungen in den entsprechenden Vorrichtungen ebenso wie diese Vorrichtungen selbst sind ein weiterer Gegenstand der vorliegenden Erfindung.

Die Erfindung durch die nachfolgenden Beispiele näher erläutert wird, ohne sie dadurch einschränken zu wollen.

### Beispiele:

Die nachfolgenden Synthesen werden, sofern nicht anders angegeben, unter einer Schutzgasatmosphäre durchgeführt. Die Edukte können von der Firma ALDRICH (Tris-(4-bromphenyl)amin, 4-tert-Butylstyrol, 4-Fluorstyrol, Palladium(II)acetat, N,N-Dimethylglycin, Anorganika, Lösemittel) und von Epsilon Chimie Products (Diethyl(4-brombenzyl)phosphonat) bezogen werden. Tris(4-formylphenyl)amin kann gemäß Synthesis 2005, 11, 1771 dargestellt werden. Tris[(phenylvinyl)phenyl]amin kann gemäß Macromolecules 1996, 29, 7705 synthetisiert werden. 4-Trifluormethoxystyrol kann gemäß J. Org. Chem. 1964, 29, 1 dargestellt werden.
6-Ethenyl-1,2,3,4-tetrahydro-1,1,4,4-tetramethyl-naphthalin kann gemäß J. Med. Chem. 1989, 32, 1504 dargestellt werden.

### Beispiel 1: Tris(phenylvinylphenyl)amin (Dotand D1)

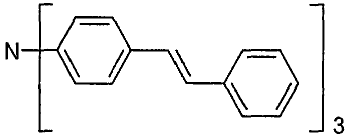

Ein Gemisch aus 24.1 g (50 mmol) Tris-(4-bromphenyl)amin, 25.8 ml (225 mmol) Styrol, 337 mg (1.5 mmol) Palladium(II)acetat, 1.55 g (15 mmol) N,N-Dimethylglycin, 81 mg (0.5 mmol) Eisen(III)chlorid und 37.8 g (450 mmol) Natriumhydrogencarbonat in 500 ml NMP wird langsam unter gutem Rühren auf 140 °C erhitzt und anschließend 16 h bei dieser Temperatur gerührt. Nach Erkalten werden 500 ml Dichlormethan und 1000 ml Wasser zugesetzt. Die organische Phase wird abgetrennt und fünfmal mit je 500 ml Wasser gewaschen. Nach Trocknen über Natriumsulfat wird die organische Phase zur Trockene eingeengt. Der so erhaltene gelbe Feststoff wird sechsmal aus Toluol umkristallisiert, dann zweimal mit je 500 ml Ethanol unter Rückfluss ausgerührt und anschließend dreimal im Vakuum (T = 350 °C, p = 5 x 10⁻⁵ mbar) sublimiert. Es werden 18.8 g (34 mmol) (entsprechend 68.1 % d. Th.) des Produkts mit einer Reinheit größer 99.5 % nach HPLC erhalten.

### Beispiel 2: Tris[4-(methylphenylvinyl)phenyl]amin (Dotand D2)

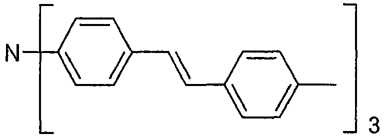

Ein Gemisch aus 24.1 g (50 mmol) Tris-(4-bromphenyl)amin, 29.6 ml (225 mmol) 4-Methylstyrol, 337 mg (1.5 mmol) Palladium(II)acetat, 1.55 g (15 mmol) N,N-Dimethylglycin, 81 mg (0.5 mmol) Eisen(III)chlorid und 37.8 g (450 mmol) Natriumhydrogencarbonat in 500 ml NMP wird langsam unter gutem Rühren auf 140 °C erhitzt und anschließend 16 h bei dieser Temperatur gerührt. Nach Erkalten werden 500 ml Dichlormethan und 1000 ml Wasser zugesetzt. Die organische Phase wird abgetrennt und fünfmal mit je 500 ml Wasser gewaschen. Nach Trocknen über Natriumsulfat wird die organische Phase zur Trockene eingeengt. Der so erhaltene gelbe Feststoff wird sechsmal aus Toluol umkristallisiert, dann zweimal mit je 500 ml Ethanol unter Rückfluss ausgerührt und anschließend dreimal im Vakuum (T = 350 °C, p = 5 x 10⁻⁵ mbar) sublimiert. Es werden 15.7 g (26 mmol) (entsprechend 52.8 % d. Th.) des Produkts mit einer Reinheit größer 99.5 % nach HPLC erhalten.

### Beispiel 3: Tris[4-(tert-butylphenylvinyl)phenyl]amin (Dotand D3)

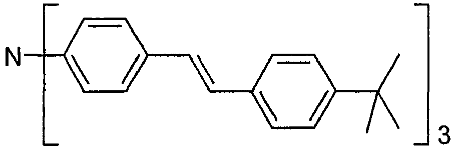

Ein Gemisch aus 24.1 g (50 mmol) Tris-(4-bromphenyl)amin, 42.4 ml (225 mmol) 4-tert-Butylstyrol, 337 mg (1.5 mmol) Palladium(II)acetat, 1.55 g (15 mmol) N,N-Dimethylglycin, 81 mg (0.5 mmol) Eisen(III)chlorid und 37.8 g (450 mmol) Natriumhydrogencarbonat in 500 ml NMP wird langsam unter gutem Rühren auf 140 °C erhitzt und anschließend 16 h bei dieser Temperatur gerührt. Nach Erkalten werden 500 ml Dichlormethan und 1000 ml Wasser zugesetzt. Die organische Phase wird abgetrennt und fünfmal mit 500 ml Wasser gewaschen. Nach Trocknen über Natriumsulfat wird die organische Phase zur Trockene eingeengt. Der so erhaltene gelbe Feststoff wird viermal aus Dioxan und zweimal aus Toluol / n-Heptan (2:1, v:v) umkristallisiert, dann zweimal mit 500 ml Ethanol unter Rückfluss ausgerührt und anschließend dreimal im Vakuum (T = 330 °C, p = 5 x 10⁻⁵ mbar) sublimiert. Es werden 17.4 g (24 mmol) (entsprechend 48.3 % d. Th.) des Produkts mit einer Reinheit größer 99.5 % nach HPLC erhalten.

### Beispiel 4: Tris(4-(trimethylsilylphenylvinyl)phenyl)amin (Dotand D4)

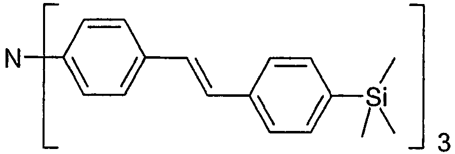

### a) Tris(4-bromphenylvinylphenyl)amin

Ein auf 0 °C gekühltes Gemisch von 179.7 g (585 mmol) Diethyl(4-brombenzyl)phosphonat und 1000 ml DMF wird unter gutem Rühren mit 112.5 g (1.17 mol) Natrium-tert-butylat versetzt. Die Mischung wird 30 min. bei 0 °C nachgerührt und dann tropfenweise mit einer Lösung von 49.6 g (150 mmol) Tris(4-formylphenyl)amin in 1000 ml DMF versetzt. Die Mischung wird 3 h bei 0 bis 5 °C nachgerührt und dann unter Eiskühlung durch Zugabe eines Gemischs aus 250 ml 2.5 M HCl, 600 ml Wasser und 200 ml Ethanol hydrolysiert. Der ausgefallene Feststoff wird abgesaugt, dreimal mit je 300 ml Ethanol / Wasser (1:1, v:v) und dreimal mit je 300 ml Ethanol gewaschen und im Vakuum getrocknet. Der Feststoff wird aus 300 ml DMF umkristallisiert, anschließend mit 500 ml Ethanol ausgekocht und im Vakuum getrocknet. Ausbeute 97.2 g (123 mmol) (entsprechend 82.1 % d. Th.) bei einer Reinheit von 99.0 % nach NMR.

### b) Tris(4-(trimethylsilylphenylvinyl)phenyl)amin

Eine gut gerührte Suspension von 39.4 g (50 mmol) Tris(4-bromphenylvinylphenyl)amin in 1000 ml Diethylether wird bei Raumtemperatur tropfenweise mit 100 ml (250 mmol) n-BuLi (2.5 M in n-Hexan) versetzt und 3 h bei Raumtemperatur nachgerührt. Die so erhaltene Lösung wird auf -78 °C abgekühlt, mit einem Gemisch aus 57.5 ml (450 mmol) Chlortrimethylsilan in 100 ml Diethylether versetzt und langsam auf Raumtemperatur erwärmt. Nach Zugabe von 500 ml Wasser wird die organische Phase abgetrennt, über Magnesiumsulfat getrocknet und eingeengt. Der so erhaltene gelbe Feststoff wird fünfmal aus Toluol / Acetonitril umkristallisiert und anschließend dreimal im Vakuum (T = 310 °C, p = 5 x 10⁻⁵ mbar) sublimiert. Es werden 23.6 g (31 mmol) (entsprechend 61.4 % d. Th.) des Produkts mit einer Reinheit größer 99.5 % nach HPLC erhalten.

### Beispiel 5: Tris[((4-boronsäurepinacolester)phenylvinyl)phenyl]amin (Dotand D5)

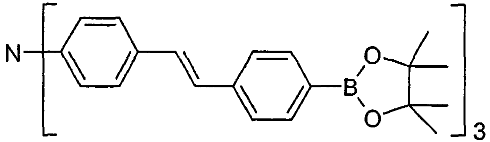

Eine gut gerührte Suspension von 39.4 g (50 mmol) Tris(4-bromphenylvinylphenyl)amin (aus Beispiel 4a) in 1000 ml Diethylether wird bei Raumtemperatur tropfenweise mit 100 ml (250 mmol) n-BuLi (2.5 M in n-Hexan) versetzt und 3 h bei Raumtemperatur nachgerührt. Die so erhaltene Lösung wird auf -78 °C abgekühlt, mit einem Gemisch aus 50.2 ml (450 mmol) Trimethylborat in 100 ml Diethylether versetzt und langsam auf Raumtemperatur erwärmt. Nach Zugabe eines Gemischs von 40 ml Essigsäure und 500 ml Wasser und 30 min. Nachrühren wird die organische Phase abgetrennt und eingeengt. Der so erhaltene gelbe Feststoff wird in 500 ml Toluol suspendiert, mit 17.7 g (150 mmol) Pinakol versetzt und bis zum Ende der Wasserabscheidung gekocht. Das nach Entfernen des Toluols erhaltene Öl wird fünfmal aus Toluol / Acetonitril umkristallisiert und anschließend dreimal im Vakuum (T = 290 °C, p = 5 x 10⁻⁵ mbar) sublimiert. Es werden 25.1 g (27 mmol) (entsprechend 54.0 % d. Th.) des Produkts mit einer Reinheit größer 99.0 % nach HPLC erhalten.

### Beispiel 6: Tris[((4-trifluormethoxy)phenylvinyl)phenyl]amin (Dotand D6)

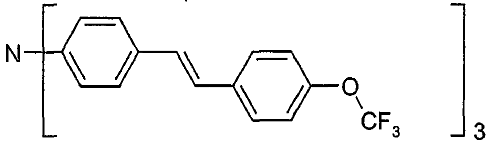

Ein Gemisch aus 24.1 g (50 mmol) Tris-(4-bromphenyl)amin, 30.2 g (225 mmol) 4-Trifluormethoxystyrol, 337 mg (1.5 mmol) Palladium(II)-acetat, 1.55 g (15 mmol) N,N-Dimethylglycin, 81 mg (0.5 mmol) Eisen(III)-chlorid und 37.8 g (450 mmol) Natriumhydrogencarbonat in 500 ml NMP wird langsam unter gutem Rühren auf 140 °C erhitzt und anschließend 24 h bei dieser Temperatur gerührt. Nach Erkalten werden 500 ml Dichlormethan und 1000 ml Wasser zugesetzt. Die organische Phase wird abgetrennt und fünfmal mit 500 ml Wasser gewaschen. Nach Trocknen über Natriumsulfat wird die organische Phase zur Trockene eingeengt. Der so erhaltene gelbe Feststoff fünfmal aus Toluol / n-Heptan (1:1, v:v) umkristallisiert, dann zweimal mit 500 ml Ethanol unter Rückfluss ausgerührt und anschließend dreimal im Vakuum (T = 280 °C, p = 5 x 10⁻⁵ mbar) sublimiert. Es werden 11.0 g (17 mmol) (entsprechend 34.3 % d. Th.) des Produkts mit einer Reinheit größer 99.5 % nach HPLC erhalten.

### Beispiel 7: Tris[4(6-ethenyl-1,2,3,4-tetrahydro-1,1,4,4-tetramethylnaphthalin)phenyl]amin (Dotand D7)

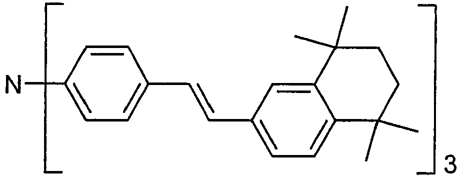

Ein Gemisch aus 24.1 g (50 mmol) Tris-(4-bromphenyl)amin, 48.2 g (225 mmol) 6-Ethenyl-1,2,3,4-tetrahydro-1,1,4,4-tetramethyl-naphthalin, 337 mg (1.5 mmol) Palladium(II)acetat, 1.55 g (15 mmol) N,N-Dimethylglycin, 81 mg (0.5 mmol) Eisen(III)chlorid und 37.8 g (450 mmol) Natriumhydrogencarbonat in 500 ml NMP wird langsam unter gutem Rühren auf 140 °C erhitzt und anschließend 16 h bei dieser Temperatur gerührt. Nach Erkalten werden 500 ml Dichlormethan und 1000 ml Wasser zugesetzt. Die organische Phase wird abgetrennt und fünfmal mit 500 ml Wasser gewaschen. Nach Trocknen über Natriumsulfat wird die organische Phase zur Trockene eingeengt. Der so erhaltene gelbe Feststoff wird fünfmal aus Dioxan umkristallisiert, dann zweimal mit 500 ml Ethanol unter Rückfluss ausgerührt und anschließend dreimal im Vakuum (T = 340 °C, p = 5 x 10⁻⁵ mbar) sublimiert. Es werden 24.7 g (28 mmol) (entsprechend 56.0 % d. Th.) des Produkts mit einer Reinheit größer 99.5 % nach HPLC erhalten.

### Beispiel 8: Tris[4-(fluorphenylvinyl)phenyl]amin (Dotand D8)

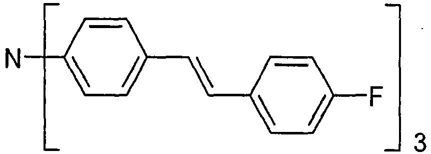

Ein Gemisch aus 24.1 g (50 mmol) Tris-(4-bromphenyl)amin, 26.8 ml (225 mmol) 4-Fluorstyrol, 337 mg (1.5 mmol) Palladium(II)acetat, 1.55 g (15 mmol) N,N-Dimethylglycin, 81 mg (0.5 mmol) Eisen(III)chlorid und 37.8 g (450 mmol) Natriumhydrogencarbonat in 500 ml NMP wird langsam unter gutem Rühren auf 140 °C erhitzt und anschließend 16 h bei dieser Temperatur gerührt. Nach Erkalten werden 500 ml Dichlormethan und 1000 ml Wasser zugesetzt. Die organische Phase wird abgetrennt und fünfmal mit 500 ml Wasser gewaschen. Nach Trocknen über Natriumsulfat wird die organische Phase zur Trockene eingeengt. Der so erhaltene gelbe Feststoff wird fünfmal aus Dioxan umkristallisiert, dann zweimal mit 500 ml Ethanol unter Rückfluss ausgerührt und anschließend dreimal im Vakuum (T = 290 °C, p = 5 x 10⁻⁵ mbar) sublimiert. Es werden 15.4 g (26 mmol) (entsprechend 56.1 % d. Th.) des Produkts mit einer Reinheit größer 99.5 % nach HPLC erhalten.

### Beispiel 9: Herstellung der OLEDs

Die Herstellung von OLEDs erfolgt nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wird. Der grundlegende Aufbau und die verwendeten Materialien (außer der emittierenden Schicht) sind in den Beispielen zur besseren Vergleichbarkeit identisch. Analog dem o. g. allgemeinen Verfahren werden OLEDs mit folgendem Aufbau erzeugt:

| | |
|---|---|
| Lochinjektionsschicht (HIL) | 20 nm PEDOT (aus Wasser aufgeschleudert; bezogen von H. C. Starck, Goslar, Deutschland; Poly(3,4-ethylendioxy-2,5-thiophen)) |
| Lochtransportschicht (HTM) | 10 nm 2,2',7,7'-Tetrakis(di-paratolylamino)spiro-9,9'-bifluoren (abgekürzt als **HTM-1**) |
| Lochtransportschicht (HTM) | 30 nm NPB (N-Naphthyl-N-phenyl-4,4'-diaminobiphenyl) |
| Emissionschicht (EML) | siehe Tabelle 1 für Materialien, Konzentration und Schichtdicke |
| Elektronenleiter (ETL) | 20 nm AlQ₃ (bezogen von SynTec, Tris(chinolinato)aluminium(III)) |
| Kathode | 1 nm LiF, darauf 150 nm Al |

Diese OLEDs werden standardmäßig charakterisiert; hierfür werden die Elektrolumineszenzspektren, die Effizienz (gemessen in cd/A), die Leistungseffizienz (gemessen in lm/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 1000 cd/m² auf die Hälfte gesunken ist.

In Tabelle 1 sind die Ergebnisse einiger OLEDs (Beispiele 10 bis 22) zusammengefasst, die die Dotanden **D1** bis **D8** (erfindungsgemäße Beispiele) und den Dotanden **V1** (Vergleichsbeispiel) enthalten, wobei jeweils die Zusammensetzung der EML inklusive der Schichtdicken mit aufgeführt ist. Die Struktur des Dotanden **V1** ist im Folgenden abgebildet:

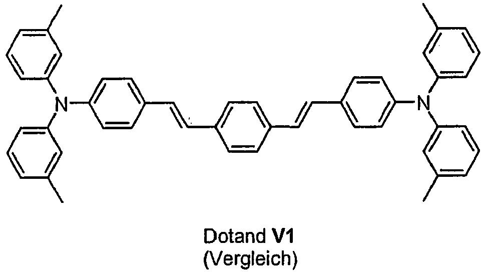

Als erfindungsgemäßes Hostmaterial werden 9,10-Bis(1-naphthyl)-anthracen (**H1**) und 9,10-Bis(2-spirobifluorenyl)anthracen (**H2**) eingesetzt. Als Vergleichs-Hostmaterial wird das Hostmaterial **H3** eingesetzt. Die Hostmaterialien sind im Folgenden abgebildet:

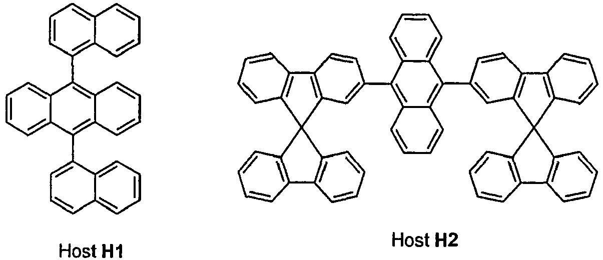

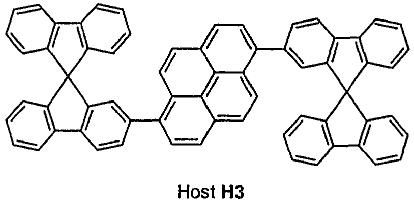

Für diese OLEDs wird dabei der Dotiergrad, also der Anteil des Dotanden im Hostmaterial, jeweils so optimiert, dass die besten Ergebnisse bezüglich Farbe und Lebensdauer erhalten werden. Mit **D1** und **D2** werden die besten Ergebnisse mit einem Dotiergrad von 2 % erhalten. Mit **D3** bis **D8** und **V1** werden die besten Ergebnisse mit einem Dotiergrad von 5 % erhalten.

Wie man den Beispielen in der Tabelle 1 entnehmen kann, zeigen die erfindungsgemäßen Elektrolumineszenzvorrichtungen tiefblaue Emission mit besseren Farbkoordinaten und verbesserte Effizienz bei deutlich verbesserter Lebensdauer im Vergleich zu den Tristilbenaminderivaten gemäß dem Stand der Technik. Zwar weist auch der Dotand **V1** gemäß dem Stand der Technik gute Lebensdauern auf. Wegen der grünblauen Emissionsfarbe ist dieser Dotand jedoch nicht in hochwertigen Produkten anwendbar. Insbesondere ist weiterhin zu beachten, dass die in den Beispielen angegebenen Lebensdauern bei gleicher Anfangshelligkeit gemessen wurden. Vergleicht man die Lebensdauern bei gleicher Stromdichte und rechnet auf vergleichbare Farbkoordinaten um, so weisen die erfindungsgemäßen Elektrolumineszenzvorrichtungen längere Lebensdauern auf als die Vorrichtungen gemäß dem Stand der Technik.

**Tabelle 1**

| **Beispiel** | **HTL1** | **HTL2** | **EML** | **Max Effizienz (cd/A)** | **Spannung (V) bei 100cd/m²** | **CIE** | **Lebensdauer (h)** |
|---|---|---|---|---|---|---|---|
| Beispiel 10 | **HTM-1** | **NPB** | **H1 : D1** (2%) | 3.4 | 5.1 | x=0.15; y=0.16 | 500 |
| | (10 nm) | (30 nm) | (30 nm) | | | | |
| Beispiel 11 | **HTM-1** | **NPB** | **H1 : D2** (2%) | 3.6 | 5.0 | x=0.15; y=0.17 | 800 |
| | (10 nm) | (30 nm) | (30 nm) | | | | |
| Beispiel 12 | **HTM-1** | **NPB** | **H1 : D3** (5%) | 4.2 | 5.1 | x=0.15; y=0.11 | 1600 |
| | (10 nm) | (20 nm) | (30 nm) | | | | |
| Beispiel 13 | **HTM-1** | **NPB** | **H1 : D4** (5%) | 4.9 | 4.5 | x=0.15; y=0.13 | 1200 |
| | (10 nm) | (30 nm) | (30 nm) | | | | |
| Beispiel 14 | **HTM-1** | **NPB** | **H1 : D5** (5%) | 4.7 | 4.7 | x=0.15; y=0.14 | 1100 |
| | (10 nm) | (30 nm) | (30 nm) | | | | |
| Beispiel 15 | **HTM-1** | **NPB** | **H1 : D6** (5%) | 4.5 | 4.9 | x=0.15; y=0.13 | 1000 |
| | (10 nm) | (30 nm) | (30 nm) | | | | |
| Beispiel 16 | **HTM-1** | **NPB** | **H1 : D7** (5%) | 4.4 | 5.0 | x=0.15; y=0.12 | 1500 |
| | (10 nm) | (20 nm) | (30 nm) | | | | |
| Beispiel 17 | **HTM-1** | **NPB** | **H1 : D8** (5%) | 4.0 | 5.2 | x=0.15; y=0.14 | 1050 |
| | (10 nm) | (30 nm) | (30 nm) | | | | |
| Beispiel 18 (Vergleich) | **HTM-1** | **NPB** | **H1 : V1** (5%) | 12.2 | 5.3 | x=0.17; y=0.33 | 7000 |
| | (10 nm) | (30 nm) | (30 nm) | | | | |
| Beispiel 19 | **HTM-1** | **NPB** | **H2 : D1** (2%) | 3.1 | 5.5 | x=0.15; y=0.14 | 370 |
| | (10 nm) | (30 nm) | (30 nm) | | | | |
| Beispiel 20 | **HTM-1** | **NPB** | **H2 : D2** (2%) | 3.3 | 5.4 | x=0.15; y=0.16 | 500 |
| | (10 nm) | (30 nm) | (30 nm) | | | | |
| Beispiel 21 | **HTM-1** | **NPB** | **H2 : D3** (5%) | 3.6 | 5.6 | x=0.15; y=0.11 | 650 |
| | (10 nm) | (30 nm) | (30 nm) | | | | |
| Beispiel 22 (Vergleich) | **HTM-1** | **NPB** | **H3: D1** (2%) | 3.0 | 5.7 | x=0.16; y=.017 | 280 |
| | (10 nm) | (30 nm) | (30 nm) | | | | |

## Patentansprüche

1. Organische Elektrolumineszenzvorrichtung, enthaltend Anode, Kathode und mindestens eine organische Schicht, **dadurch gekennzeichnet, dass** die organische Schicht die folgenden Komponenten enthält:
a) mindestens eine Verbindung der Formel (1), 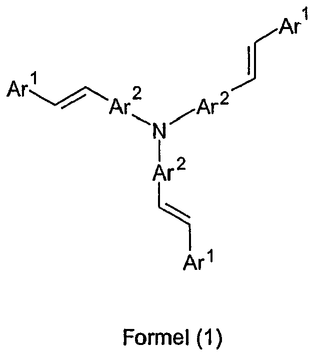 wobei für die verwendeten Symbole gilt:
Ar¹ ist gleich oder verschieden bei jedem Auftreten ein aromatisches oder heteroaromatisches Ringsystem mit 5 bis 30 aromatischen Ringatomen, welches durch einen oder mehrere Reste R¹ substituiert sein kann;
Ar² ist gleich oder verschieden bei jedem Auftreten eine bivalente Arylen- oder Heteroarylengruppe mit 5 bis 20 aromatischen Ringatomen, welche durch einen oder mehrere Reste R¹ substituiert sein kann;
R¹ ist gleich oder verschieden bei jedem Auftreten H, F, Cl, Br, I, CN, Si(R³)₃, N(R³)₂, B(OR³)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch -R³C=CR³-, -C≡C-, Si(R³)₂, Ge(R³)₂, Sn(R³)₂, C=O, C=S, C=Se, C=NR³, -O-, -S-, -N(R³)- oder -CONR³- ersetzt sein können und wobei ein oder mehrere H-Atome durch F, Cl, Br, I, CN oder NO₂ ersetzt sein können, oder eine Aryl- oder Heteroarylgruppe mit 5 bis 30 aromatischen Ringatomen, die mit einem oder mehreren Resten R³ substituiert sein kann, oder eine Aryloxy- oder Heteroaryloxygruppe mit 5 bis 24 aromatischen Ringatomen, die durch einen oder mehrere Reste R³ substituiert sein kann, oder eine Kombination aus zwei, drei, vier oder fünf dieser Systeme; dabei können auch zwei oder mehrere Substituenten R¹ miteinander ein mono- oder polycyclisches, aliphatisches oder aromatisches Ringsystem bilden;
R³ ist bei jedem Auftreten gleich oder verschieden H oder ein aliphatischer oder aromatischer Kohlenwasserstoffrest mit 1 bis 20 C-Atomen; dabei können auch zwei oder mehrere Reste R³ miteinander ein mono- oder polycyclisches, aliphatisches oder aromatisches Ringsystem bilden;
und
b) mindestens eine Verbindung der Formel (2) oder der Formel (3), 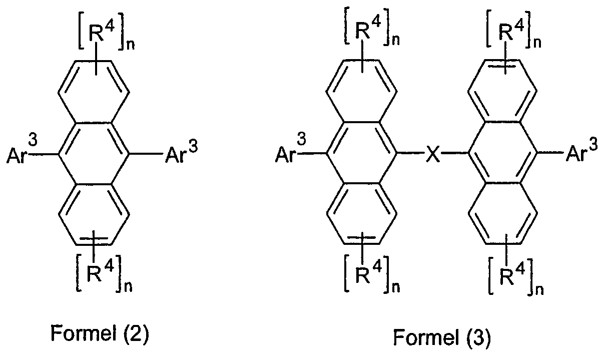 wobei für die verwendeten Symbole gilt:
Ar³ ist gleich oder verschieden bei jedem Auftreten ein aromatisches oder heteroaromatisches Ringsystem mit 5 bis 30 aromatischen Ringatomen, welches durch einen oder mehrere Reste R² substituiert sein kann;
X ist bei jedem Auftreten gleich oder verschieden eine bivalente Gruppe enthaltend 1 bis 40 C-Atome oder -O-, -S-, -NH- oder eine Einfachbindung;
R², R⁴ ist gleich oder verschieden bei jedem Auftreten H, F, Cl, Br, I, CN, N(R³)₂, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch -R³C=CR³-, -C≡C-, Si(R³)₂, Ge(R³)₂, Sn(R³)₂, C=O, C=S, C=Se, C=NR³, -O-, -S-, -N(R³)- oder -CONR³- ersetzt sein können und wobei ein oder mehrere H-Atome durch F, Cl, Br, I, CN oder NO₂ ersetzt sein können, oder eine Aryl- oder Heteroarylgruppe mit 5 bis 30 aromatischen Ringatomen, die mit einem oder mehreren Resten R³ substituiert sein kann, oder eine Aryloxy- oder Heteroaryloxygruppe mit 5 bis 24 aromatischen Ringatomen, die durch einen oder mehrere Reste R³ substituiert sein kann, oder eine Kombination aus zwei, drei, vier oder fünf dieser Systeme; dabei können auch zwei oder mehrere benachbarte Substituenten R⁴ miteinander ein mono- oder polycyclisches, aliphatisches Ringsystem bilden;
R³ hat dieselbe Bedeutung, wie oben beschrieben;
n ist gleich oder verschieden bei jedem Auftreten 0, 1, 2, 3 oder 4.

2. Organische Elektrolumineszenzvorrichtung nach Anspruch 1, **dadurch gekennzeichnet, dass** die Vorrichtung mindestens eine Verbindung gemäß Formel (1a) enthält, 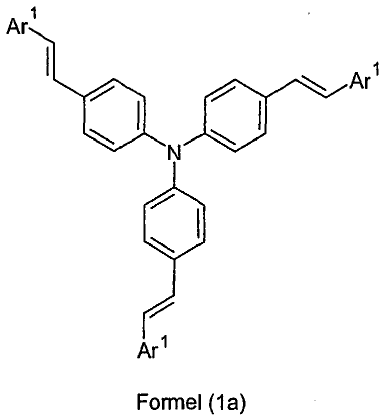 wobei Ar¹ dieselbe Bedeutung hat, wie in Anspruch 1 beschrieben, und die Phenylengruppen noch durch einen oder mehrere Reste R¹ substituiert sein können.

3. Organische Elektrolumineszenzvorrichtung nach Anspruch 1 oder 2, **dadurch gekennzeichnet, dass** das Symbol Ar¹, gleich oder verschieden bei jedem Auftreten, für eine Aryl- oder Heteroarylgruppe, insbesondere eine Arylgruppe, mit 6 bis 14 C-Atomen steht, welche durch einen oder mehrere Reste R¹ substituiert sein kann.

4. Organische Elektrolumineszenzvorrichtung nach einem oder mehreren der Ansprüche 1 bis 3, **dadurch gekennzeichnet, dass** das Symbol Ar¹ in Formel (1) bzw. Formel (1a) für eine Phenyl-, 1-Naphthyl- oder 2-Naphthylgruppe, die jeweils durch einen oder mehrere Reste R¹ substituiert sein kann, steht.

5. Organische Elektrolumineszenzvorrichtung nach einem oder mehreren der Ansprüche 1 bis 4, **dadurch gekennzeichnet, dass** die Reste R¹ an Ar¹ in para-Position zur Doppelbindung gebunden sind.

6. Organische Elektrolumineszenzvorrichtung nach einem oder mehreren der Ansprüche 1 bis 5, **dadurch gekennzeichnet, dass** die Reste R¹ ausgewählt sind aus der Gruppe bestehend aus H, F, Si(R³)₃, B(OR³)₂, geradkettigen Alkyl- oder Alkoxygruppen mit 1 bis 6 C-Atomen oder verzweigten oder cyclischen Alkyl- oder Alkoxygruppen mit 3 bis 10 C-Atomen, wobei jeweils ein oder mehrere CH₂-Gruppen durch -R³C=CR³-, Si(R³)₂, -O-, -S- oder -N(R³)- ersetzt sein können und wobei jeweils ein oder mehrere H-Atome durch F ersetzt sein können, oder Aryl- oder Heteroarylgruppen mit 5 bis 14 aromatischen Ringatomen, oder einer Kombination aus zwei oder drei dieser Systeme.

7. Organische Elektrolumineszenzvorrichtung nach einem oder mehreren der Ansprüche 1 bis 6, **dadurch gekennzeichnet, dass** die Verbindungen gemäß Formel (1) eine dreizählige Symmetrieachse aufweisen.

8. Organische Elektrolumineszenzvorrichtung nach einem oder mehreren der Ansprüche 1 bis 7, **dadurch gekennzeichnet, dass** das Symbol Ar³, gleich oder verschieden bei jedem Auftreten, für ein aromatisches oder heteroaromatisches Ringsystem mit 10 bis 25 aromatischen Ringatomen steht, welches durch einen oder mehrere Reste R² substituiert sein kann.

9. Organische Elektrolumineszenzvorrichtung nach einem oder mehreren der Ansprüche 1 bis 8, **dadurch gekennzeichnet, dass** das Symbol Ar³, gleich oder verschieden bei jedem Auftreten, für eine 1-Naphthyl-, 2-Naphthyl-, 9-Anthryl-, 2-Phenanthrenyl-, 9-Phenanthrenyl-, Chinolinyl-, Isochinolinyl-, ortho-Biphenyl-, 2-Fluorenyl- oder 2-Spirobifluorenylgruppe steht, die jeweils mit einem oder mehreren Resten R² substituiert sein kann

10. Organische Elektrolumineszenzvorrichtung nach einem oder mehreren der Ansprüche 1 bis 9, **dadurch gekennzeichnet, dass** das Symbol R² in Formel (2) bzw. Formel (3), gleich oder verschieden bei jedem Auftreten, für H, F, B(OR³)₂, eine geradkettige Alkyl- oder Alkoxygruppe mit 1 bis 6 C-Atomen oder eine verzweigte oder cyclische Alkyl- oder Alkoxygruppe mit 3 bis 10 C-Atomen, wobei jeweils ein oder mehrere CH₂-Gruppen durch -R³C=CR³-, Si(R³)₂, -O-, -S- oder -N(R³)- ersetzt sein können und wobei jeweils ein oder mehrere H-Atome durch F ersetzt sein können, oder eine Aryl- oder Heteroarylgruppe mit 5 bis 14 aromatischen Ringatomen, oder eine Kombination aus zwei oder drei dieser Systeme steht; dabei können auch zwei oder mehrere benachbarte Reste R² miteinander ein mono- oder polycyclisches, aliphatisches oder aromatisches Ringsystem bilden.

11. Organische Elektrolumineszenzvorrichtung nach einem oder mehreren der Ansprüche 1 bis 10, **dadurch gekennzeichnet, dass** das Symbol X in Formel (3), gleich oder verschieden bei jedem Auftreten, eine lineare Alkylengruppe mit 1 bis 10 C-Atomen oder eine verzweigte oder cyclische Alkylen- oder Alkylidengruppe mit 3 bis 10 C-Atomen, eine bivalente aromatische Gruppe mit 6 bis 25 C-Atomen, -O-, -S- oder eine Gruppe der Formel -N(R³)- oder -P(=O)R³- oder eine Einfachbindung darstellt.

12. Organische Elektrolumineszenzvorrichtung nach einem oder mehreren der Ansprüche 1 bis 11, **dadurch gekennzeichnet, dass** die Verbindungen gemäß Formel (2) bzw. Formel (3) reine Kohlenwasserstoffverbindungen sind.

13. Organische Elektrolumineszenzvorrichtung nach einem oder mehreren der Ansprüche 1 bis 12, **dadurch gekennzeichnet, dass** außer Kathode, Anode und der emittierenden Schicht bzw. den emittierenden Schichten noch weitere Schichten enthalten sind, ausgewählt aus Lochinjektionsschicht, Lochtransportschicht, Elektronentransportschicht und/oder Elektroneninjektionsschicht, die auch dotiert sein können, und dass gegebenenfalls auch mehrere emittierenden Schichten vorhanden sein können, so dass insgesamt weiße Emission resultiert.

14. Verfahren zur Herstellung organischer Elektrolumineszenzvorrichtungen nach einem oder mehreren der Ansprüche 1 bis 13, **dadurch gekennzeichnet, dass** mindestens eine Verbindung gemäß Formel (1) zusammen mit mindestens einer Verbindung gemäß Formel (2) und/oder Formel (3) durch ein Sublimationsverfahren oder aus Lösung, beispielsweise durch ein Druckverfahren, aufgebracht werden.

15. Mischungen enthaltend mindestens eine Verbindung gemäß Formel (1) nach Anspruch 1 und mindestens eine Verbindung gemäß Formel (2) oder Formel (3) nach Anspruch 1.

16. Verwendung von Mischungen nach Anspruch 15 zur Herstellung organischer elektronischer Vorrichtungen, insbesondere organischer Elektrolumineszenzvorrichtungen.

17. Organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), organische Photorezeptoren oder organische Laserdioden (O-Laser), enthaltend mindestens eine Mischung nach Anspruch 15.

## Claims

1. Organic electroluminescent device comprising anode, cathode and at least one organic layer, **characterised in that** the organic layer comprises the following components:
a) at least one compound of the formula (1), 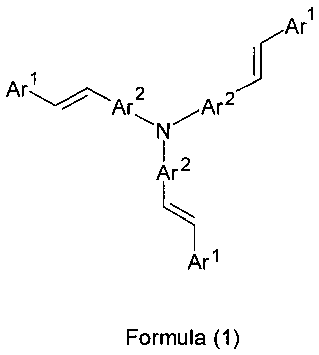 where the following applies to the symbols used:
Ar¹ is, identically or differently on each occurrence, an aromatic or heteroaromatic ring system having 5 to 30 aromatic ring atoms, which may be substituted by one or more radicals R¹;
Ar² is, identically or differently on each occurrence, a divalent arylene or heteroarylene group having 5 to 20 aromatic ring atoms, which may be substituted by one or more radicals R¹;
R¹ is, identically or differently on each occurrence, H, F, Cl, Br, I, CN, Si(R³)₃, N(R³)₂, B(OR³)₂, a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R³, where one or more non-adjacent CH₂ groups may be replaced by -R³C=CR³-, -C=C-, Si(R³)₂, Ge(R³)₂, Sn(R³)₂, C=O, C=S, C=Se, C=NR³, -O-, -S-, -N(R³)- or -CONR³- and where one or more H atoms may be replaced by F, Cl, Br, I, CN or NO₂, or an aryl or heteroaryl group having 5 to 30 aromatic ring atoms, which may be substituted by one or more radicals R³, or an aryloxy or heteroaryloxy group having 5 to 24 aromatic ring atoms, which may be substituted by one or more radicals R³, or a combination of two, three, four or five of these systems; two or more substituents R¹ here may also form a mono- or polycyclic, aliphatic or aromatic ring system with one another;
R³ is on each occurrence, identically or differently, H or an aliphatic or aromatic hydrocarbon radical having 1 to 20 C atoms; two or more radicals R³ here may also form a mono- or polycyclic, aliphatic or aromatic ring system with one another;
and
b) at least one compound of the formula (2) or of the formula (3), 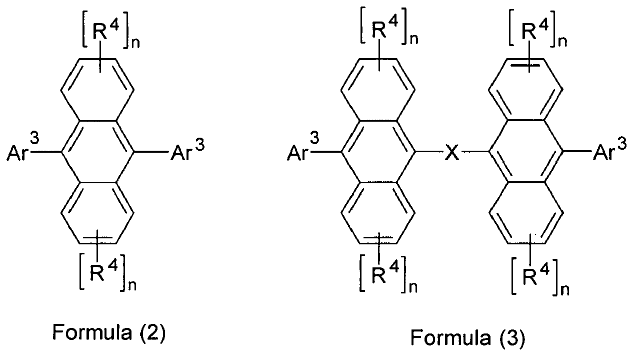 where the following applies to the symbols used:
Ar³ is, identically or differently on each occurrence, an aromatic or heteroaromatic ring system having 5 to 30 aromatic ring atoms, which may be substituted by one or more radicals R²;
X is on each occurrence, identically or differently, a divalent group containing 1 to 40 C atoms or -O-, -S-, -NH- or a single bond;
R², R⁴ are, identically or differently on each occurrence, H, F, Cl, Br, I, CN, N(R³)₂, a straight-chain alkyl, alkoxy or thioalkoxy group having 1 to 40 C atoms or a branched or cyclic alkyl, alkoxy or thioalkoxy group having 3 to 40 C atoms, each of which may be substituted by one or more radicals R³, where one or more non-adjacent CH₂ groups may be replaced by -R³C=CR³-, -C=C-, Si(R³)₂, Ge(R³)₂, Sn(R³)₂, C=O, C=S, C=Se, C=NR³, -O-, -S-, -N(R³)- or -CONR³- and where one or more H atoms may be replaced by F, Cl, Br, I, CN or NO₂, or an aryl or heteroaryl group having 5 to 30 aromatic ring atoms, which may be substituted by one or more radicals R³, or an aryloxy or heteroaryloxy group having 5 to 24 aromatic ring atoms, which may be substituted by one or more radicals R³, or a combination of two, three, four or five of these systems; two or more adjacent substituents R⁴ here may also form a mono- or polycyclic, aliphatic ring system with one another;
R³ has the same meaning as described above;
n is, identically or differently on each occurrence, 0, 1, 2, 3 or 4.

2. Organic electroluminescent device according to Claim 1, **characterised in that** the device comprises at least one compound of the formula (1 a), 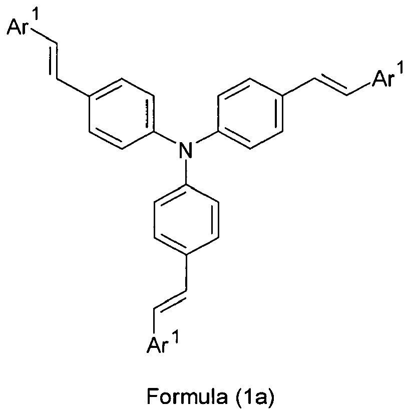 where Ar¹ has the same meaning as described in Claim 1 and the phenylene groups may also be substituted by one or more radicals R¹.

3. Organic electroluminescent device according to Claim 1 or 2, **characterised in that** the symbol Ar¹ stands, identically or differently on each occurrence, for an aryl or heteroaryl group, in particular an aryl group, having 6 to 14 C atoms, which may be substituted by one or more radicals R¹.

4. Organic electroluminescent device according to one or more of Claims 1 to 3, **characterised in that** the symbol Ar¹ in formula (1) or formula (1a) stands for a phenyl, 1-naphthyl or 2-naphthyl group, each of which may be substituted by one or more radicals R¹.

5. Organic electroluminescent device according to one or more of Claims 1 to 4, **characterised in that** the radicals R¹ are bonded to Ar¹ in the para-position to the double bond.

6. Organic electroluminescent device according to one or more of Claims 1 to 5, **characterised in that** the radicals R¹ are selected from the group consisting of H, F, Si(R³)₃, B(OR³)₂, straight-chain alkyl or alkoxy groups having 1 to 6 C atoms or branched or cyclic alkyl or alkoxy groups having 3 to 10 C atoms, where in each case one or more CH₂ groups may be replaced by -R³C=CR³-, Si(R³)₂, -O-, -S- or -N(R³)- and where in each case one or more H atoms may be replaced by F, or aryl or heteroaryl groups having 5 to 14 aromatic ring atoms, or a combination of two or three of these systems.

7. Organic electroluminescent device according to one or more of Claims 1 to 6, **characterised in that** the compounds of the formula (1) have a threefold axis of symmetry.

8. Organic electroluminescent device according to one or more of Claims 1 to 7, **characterised in that** the symbol Ar³ stands, identically or differently on each occurrence, for an aromatic or heteroaromatic ring system having 10 to 25 aromatic ring atoms, which may be substituted by one or more radicals R².

9. Organic electroluminescent device according to one or more of Claims 1 to 8, **characterised in that** the symbol Ar³ stands, identically or differently on each occurrence, for a 1-naphthyl, 2-naphthyl, 9-anthryl, 2-phenanthrenyl, 9-phenanthrenyl, quinolinyl, isoquinolinyl, ortho-biphenyl, 2-fluorenyl or 2-spirobifluorenyl group, each of which may be substituted by one or more radicals R².

10. Organic electroluminescent device according to one or more of Claims 1 to 9, **characterised in that** the symbol R² in formula (2) or formula (3) stands, identically or differently on each occurrence, for H, F, B(OR³)₂, a straight-chain alkyl or alkoxy group having 1 to 6 C atoms or a branched or cyclic alkyl or alkoxy group having 3 to 10 C atoms, where in each case one or more CH₂ groups may be replaced by -R³C=CR³-, Si(R³)₂, -O-, -S- or -N(R³)- and where in each case one or more H atoms may be replaced by F, or an aryl or heteroaryl group having 5 to 14 aromatic ring atoms, or a combination of two or three of these systems; two or more adjacent radicals R² here may also form a mono- or polycyclic, aliphatic or aromatic ring system with one another.

11. Organic electroluminescent device according to one or more of Claims 1 to 10, **characterised in that** the symbol X in formula (3) represents, identically or differently on each occurrence, a linear alkylene group having 1 to 10 C atoms or a branched or cyclic alkylene or alkylidene group having 3 to 10 C atoms, a divalent aromatic group having 6 to 25 C atoms, -O-, -S- or a group of the formula -N(R³)- or -P(=O)R³- or a single bond.

12. Organic electroluminescent device according to one or more of Claims 1 to 11, **characterised in that** the compounds of the formula (2) or formula (3) are pure hydrocarbon compounds.

13. Organic electroluminescent device according to one or more of Claims 1 to 12, **characterised in that**, apart from the cathode, anode and emitting layer or emitting layers, further layers are also present, selected from hole-injection layer, hole-transport layer, electron-transport layer and/or electron-injection layer, which may also be doped, and **in that**, if desired, a plurality of emitting layers may also be present, resulting overall in white emission.

14. Process for the production of organic electroluminescent devices according to one or more of Claims 1 to 13, **characterised in that** at least one compound of the formula (1) is applied together with at least one compound of the formula (2) and/or formula (3) by a sublimation method or from solution, for example by a printing process.

15. Mixtures comprising at least one compound of the formula (1) according to Claim 1 and at least one compound of the formula (2) or formula (3) according to Claim 1.

16. Use of mixtures according to Claim 15 for the production of organic electronic devices, in particular organic electroluminescent devices.

17. Organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), organic photoreceptors or organic laser diodes (O-lasers) comprising at least one mixture according to Claim 15.

## Revendications

1. Dispositif électroluminescent organique comprenant une anode, une cathode et au moins une couche organique, **caractérisé en ce que** la couche organique comprend les componsants qui suivent :
a) au moins un composé de la formule (1) : 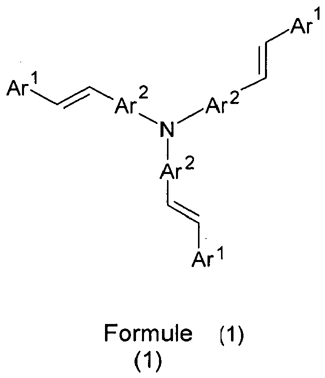 dans laquelle ce que suit s'applique aux symboles utilisés :
Ar¹ est, de manière identique ou différente pour chaque occurrence, un système de cycle aromatique ou hétéroaromatique comportant 5 à 30 atomes de cycle aromatique, lequel peut être substitué par un radical ou plusieurs radicaux R¹ ;
Ar² est, de manière identique ou différente pour chaque occurrence, un groupe arylène ou hétéroarylène divalent comportant 5 à 20 atomes de cycle aromatique, lequel peut être substitué par un radical ou plusieurs radicaux R¹ ;
R¹ est, de manière identique ou différente pour chaque occurrence, H, F, CI, Br, I, CN, Si(R³)₃, N(R³)₂, B(OR³)₂, un groupe alkyle, alcoxy ou thioalcoxy en chaîne droite comportant 1 à 40 atome(s) de C ou un groupe alkyle, alcoxy ou thioalcoxy ramifié ou cyclique comportant 3 à 40 atomes de C, dont chacun peut être substitué par un radical ou plusieurs radicaux R³, où un ou plusieurs groupe(s) CH₂ non adjacents peut/peuvent être remplacé(s) par -R³C=CR³-, -C=C-, Si(R³)₂, Ge(R³)₂, Sn(R³)₂, C=O, C=S, C=Se, C=NR³, -O-, -S-, -N(R³)- ou -CONR³- et où un ou plusieurs atome(s) de H peut/ peuvent être remplacé(s) par F, Cl, Br, I, CN ou NO₂, ou un groupe aryle ou hétéroaryle comportant 5 à 30 atomes de cycle aromatique, lequel peut être substitué par un radical ou plusieurs radicaux R³, ou un groupe aryloxy ou hétéroaryloxy comportant 5 à 24 atomes de cycle aromatique, lequel peut être substitué par un radical ou plusieurs radicaux R³, ou une combinaison de deux, trois, quatre ou cinq de ces systèmes ; deux substituants R¹ ou plus peuvent ici également former un système de cycle aliphatique ou aromatique monocyclique ou polycyclique l'un avec l'autre ou les uns avec les autres ;
R³ est, pour chaque occurrence, de manière identique ou différente, H ou un radical hydrocarbone aliphatique ou aromatique comportant 1 à 20 atome(s) de C ; deux radicaux R³ ou plus peuvent ici également former un système de cycle aliphatique ou aromatique monocyclique ou polycyclique l'un avec l'autre ou les uns avec les autres ;
et
b) au moins un composé de la formule (2) ou de la formule (3) : 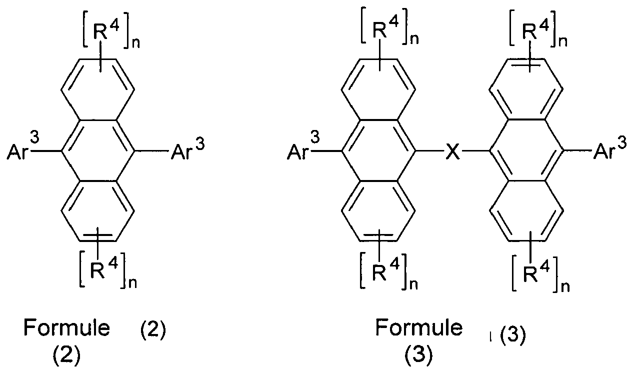 dans lesquelles ce qui suit s'applique aux symboles utilisés :
Ar³ est, de manière identique ou différenté pour chaque occurrence, un système de cycle aromatique ou hétéroaromatique comportant 5 à 30 atomes de cycle aromatique, lequel peut être substitué par un radical ou plusieurs radicaux R² ;
X est, pour chaque occurrence, de manière identique ou différente, un groupe divalent contenant 1 à 40 atome(s) de C ou -O-, -S-, -NH- ou une liaison simple ;
R², R⁴ sont, de manière identique ou différente pour chaque occurrence, H, F, CI, Br, I, CN, N(R³)₂, un groupe alkyle, alcoxy ou thioalcoxy en chaîne droite comportant 1 à 40 atome(s) de C ou un groupe alkyle, alcoxy ou thioalcoxy ramifié ou cyclique comportant 3 à 40 atomes de C, dont chacun peut être substitué par un radical ou plusieurs radicaux R³, où un ou plusieurs groupe(s) CH₂ non adjacents peut/peuvent être remplacé(s) par -R³C=CR³-, -C≡C-, Si(R³)₂, Ge(R³)₂, Sn(R³)₂, C=O, C=S, C=Se, C=NR³, -O-, -S-, -N(R³)- ou -CONR³- et où un ou plusieurs atome(s) de H peut/peuvent être remplacé(s) par F, CI, Br, I, CN ou NO₂, ou un groupe aryle ou hétéroaryle comportant 5 à 30 atomes de cycle aromatique, lequel peut être substitué par un radical ou plusieurs radicaux R³, ou un groupe aryloxy ou hétéroaryloxy comportant 5 à 24 atomes de cycle aromatique, lequel peut être substitué par un radical ou plusieurs radicaux R³, ou une combinaison de deux, trois, quatre ou cinq de ces systèmes ; deux substituants R⁴ adjacents ou plus peuvent ici également former un système de cycle aliphatique monocyclique ou polycyclique l'un avec l'autre ou les uns avec les autres ;
R³ présente la même signification que décrit ci avant ;
n est, de manière identique ou différente pour chaque occurrence, 0, 1, 2, 3 ou 4.

2. Dispositif électroluminescent organique selon la revendication 1, **caractérisé en ce que** le dispositif comprend au moins un composé de la formule (1a) : 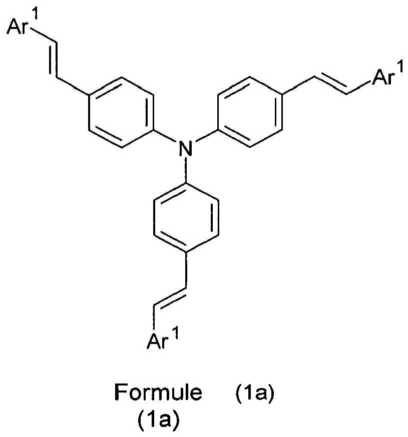 dans laquelle Ar¹ présente la même signification que décrit selon la revendication 1 et les groupes phénylène peuvent également être substitués par un radical ou plusieurs radicaux R¹.

3. Dispositif électroluminescent organique selon la revendication 1 ou 2, **caractérisé en ce que** le symbole Ar¹ représente, de manière identique ou différente pour chaque occurrence, un groupe aryle ou hétéroaryle, en particulier un groupe aryle, comportant 6 à 14 atomes de C, lequel peut être substitué par un radical ou plusieurs radicaux R¹.

4. Dispositif électroluminescent organique selon une ou plusieurs des revendications 1 à 3, **caractérisé en ce que** le symbole Ar¹ dans la formule (1) ou la formule (1a) représente un groupe phényle, 1-naphtyle ou 2-naphtyle, dont chacun peut être substitué par un radical ou plusieurs radicaux R¹.

5. Dispositif électroluminescent organique selon une ou plusieurs des revendications 1 à 4, **caractérisé en ce que** les radicaux R¹ sont liés à Ar¹ au niveau de la position para sur la liaison double.

6. Dispositif électroluminescent organique selon une ou plusieurs des revendications 1 à 5, **caractérisé en ce que** les radicaux R¹ sont choisis parmi le groupe constitué par H, F, Si(R³)₃, B(OR³)₂, des groupes alkyle ou alcoxy en chaîne droite comportant 1 à 6 atome(s) de C ou des groupes alkyle ou alcoxy ramifiés ou cycliques comportant 3 à 10 atomes de C, où, dans chaque cas, un ou plusieurs groupe(s) CH₂ peut/peuvent être remplacé(s) par -R³C=CR³-, Si(R³)₂, -O-, -S- ou -N(R³)- et où, dans chaque cas, un ou plusieurs atome(s) de H peut/ peuvent être remplacé(s) par F, ou des groupes aryle ou hétéroaryle comportant 5 à 14 atomes de cycle aromatique, ou une combinaison de deux ou trois de ces systèmes.

7. Dispositif électroluminescent organique selon une ou plusieurs des revendications 1 à 6, **caractérisé en ce que** les composés de la formule (1) présentent un axe de symétrie ternaire.

8. Dispositif électroluminescent organique selon une ou plusieurs des revendications 1 à 7, **caractérisé en ce que** le symbole Ar³ représente, de manière identique ou différente pour chaque occurrence, un système de cycle aromatique ou hétéroaromatique comportant 10 à 25 atomes de cycle aromatique, lequel peut être substitué par un radical ou plusieurs radicaux R².

9. Dispositif électroluminescent organique selon une ou plusieurs des revendications 1 à 8, **caractérisé en ce que** le symbole Ar³ représente, de manière identique ou différente pour chaque occurrence, un groupe 1-naphtyle, 2-naphtyle, 9-anthryle, 2-phénanthrényle, 9-phénanthrényle, quinolinyle, isoquinolinyle, orthobiphényle, 2-fluorényle ou 2-spirobifluorénylé, dont chacun peut être substitué par un radical ou plusieurs radicaux R².

10. Dispositif électroluminescent organique selon une ou plusieurs des revendications 1 à 9, **caractérisé en ce que** le symbole R² dans la formule (2) ou la formule (3) représente, de manière identique ou différente pour chaque occurrence, H, F, B(OR³)₂, un groupe alkyle ou alcoxy en chaîne droite comportant 1 à 6 atome(s) de C ou un groupe alkyle ou alcoxy ramifié ou cyclique comportant 3 à 10 atomes de C, où, dans chaque cas, un ou plusieurs groupe(s) CH₂ peut/peuvent être remplacé(s) par -R³C=CR³-, Si(R³)₂, -O-, -S- ou -N(R³)- et où, dans chaque cas, un ou plusieurs atome(s) de H peut/ peuvent être remplacé(s) par F, ou un groupe aryle ou hétéroaryle comportant 5 à 14 atomes de cycle aromatique, ou une combinaison de deux ou trois de ces systèmes ; deux radicaux R² adjacents ou plus peuvent ici également former un système de cycle aliphatique ou aromatique monocyclique ou polycyclique l'un avec l'autre ou les uns avec les autres.

11. Dispositif électroluminescent organique selon une ou plusieurs des revendications 1 à 10, **caractérisé en ce que** le symbole X dans la formule (3) représente, de manière identique ou différente pour chaque occurrence, un groupe alkylène linéaire comportant 1 à 10 atome(s) de C ou un groupe alkylène ou alkylidène ramifié ou cyclique comportant 3 à 10 atomes de C, un groupe aromatique divalent comportant 6 à 25 atomes de C, -O-, -S- ou un groupe de la formule -N(R³)- ou -P(=O)R³- ou une liaison simple.

12. Dispositif électroluminescent organique selon une ou plusieurs des revendications 1 à 11, **caractérisé en ce que** les composés de la formule (2) ou de la formule (3) sont des composés hydrocarbone purs.

13. Dispositif électroluminescent organique selon une ou plusieurs des revendications 1 à 12, **caractérisé en ce que**, indépendamment de la cathode, de l'anode et de la couche d'émission ou des couches d'émission, d'autres couches sont également présentes, choisies parmi une couche d'injection de trous, une couche de transport de trous, une couche de transport d'électrons et/ou une couche d'injection d'électrons, lesquelles couches peuvent également être dopées, et **en ce que**, si souhaité, les couches d'une pluralité de couches d'émission peuvent également être présentes, le résultat global étant une émission de blanc.

14. Procédé pour la fabrication de dispositifs électroluminescents organiques selon une ou plusieurs des revendications 1 à 13, **caractérisé en ce qu'**au moins un composé de la formule (1) est appliqué en association avec au moins un composé de la formule (2) et/ou de la formule (3) au moyen d'une méthode par sublimation ou à partir d'une solution, par exemple au moyen d'un processus d'impression.

15. Mélanges comprenant au moins un composé de la formule (1) selon la revendication 1 et au moins un composé de la formule (2) ou de la formule (3) selon la revendication 1.

16. Utilisation de mélanges selon la revendication 15 pour la fabrication de dispositifs électroniques organiques, en particulier de dispositifs électroluminescents organiques.

17. Transistors à effet de champ organiques (O-FET), transistors à film mince organiques (O-TFT), transistors à émission de lumière organiques (O-LET), circuits intégrés organiques (O-IC), cellules solaires organiques (O-SC), dispositifs à extinction de champ organiques (O-FQD), photorécepteurs organiques ou diodes laser organiques (O-laser) comprenant au moins un mélange selon la revendication 15.
